(19) Europäisches Patentamt
European Patent Office
Office européen des brevets

(11) **EP 2 958 489 B1**

(12) **EUROPÄISCHE PATENTSCHRIFT**

(45) Veröffentlichungstag und Bekanntmachung des Hinweises auf die Patenterteilung:
**04.10.2023 Patentblatt 2023/40**

(21) Anmeldenummer: **14706294.7**

(22) Anmeldetag: **21.02.2014**

(51) Internationale Patentklassifikation (IPC):
**A61B 5/0537** (2021.01) **A61B 5/33** (2021.01)
**A61B 5/282** (2021.01) **A61B 5/0535** (2021.01)
A61B 5/01 (2006.01) A61B 5/024 (2006.01)
A61B 5/0285 (2006.01) A61B 5/0295 (2006.01)
A61B 5/022 (2006.01) A61B 5/11 (2006.01)
A61B 5/252 (2021.01)

(52) Gemeinsame Patentklassifikation (CPC):
**A61B 5/0535; A61B 5/0537; A61B 5/282;**
**A61B 5/33;** A61B 5/01; A61B 5/02028;
A61B 5/02233; A61B 5/02416; A61B 5/0285;
A61B 5/0295; A61B 5/1102; A61B 5/252;
A61B 5/35; A61B 5/4878; A61B 5/6823; (Forts.)

(86) Internationale Anmeldenummer:
**PCT/EP2014/053384**

(87) Internationale Veröffentlichungsnummer:
**WO 2014/128237 (28.08.2014 Gazette 2014/35)**

(54) **EKG-GERÄT**

EKG DEVICE

APPAREIL ECG

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorität: **22.02.2013 AT 1352013**

(43) Veröffentlichungstag der Anmeldung:
**30.12.2015 Patentblatt 2015/53**

(73) Patentinhaber: **Skrabal, Falko**
**8043 Graz (AT)**

(72) Erfinder: **Skrabal, Falko**
**8043 Graz (AT)**

(74) Vertreter: **Schwarz & Partner Patentanwälte**
**GmbH**
**Patentanwälte**
**Wipplingerstraße 30**
**1010 Wien (AT)**

(56) Entgegenhaltungen:
EP-A2- 1 275 342 EP-A2- 2 319 411
WO-A1-2004/030535 WO-A1-2007/045006
WO-A1-2012/092303 DE-A1- 10 249 863
DE-A1-102009 035 018 DE-U1-202008 014 621
FR-A1- 2 971 137 US-A- 4 646 747
US-A1- 2005 273 015 US-A1- 2011 245 688

(52) Gemeinsame Patentklassifikation (CPC): (Forts.)
A61B 5/6824; A61B 5/6828; A61B 5/6829;
A61B 5/6831; A61B 5/6833; A61B 5/6834;
A61B 5/6838; A61B 2562/0219

**Beschreibung**

Die Erfindung betrifft ein EKG-Gerät zumindest mit Extremitätenelektroden.

Es gab bereits zahlreiche Versuche mit äußeren Abnehmern die mechanische Tätigkeit des Herzens bzw. die Funktion der Gefäße aufzuzeichnen. Ein Hauptgrund dafür, dass sich diese Methoden nicht breit durchgesetzt haben, ist, dass diese nicht hinreichend genau waren und andererseits von den Versicherungsträgern nicht refundiert werden, bzw, dass die Ärzte kaum Zeit haben, zusätzliche nicht finanzierte Untersuchungsmethoden in ihre Praxis oder in das Krankenhaus einzuführen.

Die Aufgabe der vorliegenden Erfindung ist es, die Nachteile der fehlenden Akzeptanz obiger Untersuchungen, deren mangelnde finanzielle Abgeltung durch Versicherungsträger und des zusätzlich erforderlichen Arbeitsaufwandes des medizinischen Personals durch Bereitstellen eines Vielkanal-EKG-Geräts zu überwinden, mit dem zusätzlich zu herkömmlichen EKG-Messungen die mechanische Tätigkeit des Herzens und/oder Funktionen von Gefäßen des menschlichen Körpers ohne spezifische Aktivitäten der ausführenden Person und unbemerkt vom untersuchten Patienten erfasst werden können. In dem Patent AT 502921 wurde bereits ein erster Schritt in dieser Richtung gesetzt, die vorliegende Erfindung bringt gegenüber der AT 502921 jedoch weitere signifikante Verbesserungen. Das Ziel der vorliegenden Erfindung ist es, möglichst nur die herkömmlichen EKG-Elektroden für zahlreiche weitere Ziele, so z.B. für die Mehrfrequenzimpedanzanalyse und ihrer Teilkomponenten Wirkwiderstand, Blindwiderstand und Phasenwinkel bei verschiedenen Frequenzen, bzw. die Änderung der Impedanz mit der Herzaktivität (z.B. z0, dZ, dZ/dtmax) zu verwenden und weiters für mechanische, akustische, optische und Temperaturmessungen heranzuziehen und mit möglichst wenigen zusätzlichen Elektroden auszukommen. Mittels zusätzlich an den Elektroden angebrachten Aufnehmern werden gemäß der Erfindung z.B. Phonocardiogramme, Apexocardiogramme, Pulswellenlaufzeiten, und/oder Pulswellenanalysen gemessen und aufgezeichnet. Weiters kann die Messung der Sauerstoffsättigung, der Kreislaufzeiten, wie z.B. PEP, LVET, A2O und eine genaue Erfassung der Körperkompartimente mittels segmentaler Impedanzanalyse usw. vorgenommen werden.

Die Kombination eines Phonocardiogrammaufnehmers mit einer Saugvorrichtung ist seit Jahrzehnten bekannt (z.B. Bertrand CA et al. Circulation 8: 49-57, 1956 und auch die Kombination von EKG Aufnehmer mit Phonocardiogrammaufnehmer zumindest seit 1986 (Little, US4628939, 1986).So wurde auch ein elektronisches Stethoskop beschrieben, wobei an den Rändern der Glocke die Elektroden für das EKG liegen (Watson, US20010030077, 2001). Baumer beschreibt in US 7110804 eine kombinierte EKG Elektrode mit Phonoaufnehmer in einem Hohlraum unter Verwendung eines leitenden Gels. In der Anmeldung Bauer, WO 2006020764 A3, wird eine Saugglocke beschrieben, in der ein Akustisch-zu Elektrisch Transducer positioniert wird.

Die WO 2008/031030 (Bartnik) offenbart die Gewinnung systolischer Zeitintervalle, indem von einer ersten Kurvenform, welche aus einem Impedanzsignal stammt, eine zweite Kurvenform subtrahiert wird, die aus Echokardiographie oder aus der Pulswelle oder dem Pulsoximeter gewonnen wird.

Die US 2005/0033190 (Bauer) beschreibt ein Multiaxialaccelerometer in einer EKG-Elektrode. Die US 2005/0273015 (Bauer) beschreibt eine Vakuumkammer für ein Mikrofon einer EKG-Elektrode. Die US 2009/0227886 (Bauer) beschreibt eine kontinuierliche "vibratory" Stimulation nahe der Resonanzfrequenz eines akustischen Sensors. Das US Design Patent US D675738 beschreibt ein Elektrodendesign, bei dem das Mikrofon von einer Elektrode getrennt werden kann.

Die WO 2006063255A2 (Bernstein) offenbart, aus dem Impedanzsignal über dem Thorax oder über der Brachialarterie das Schlagvolumen zu bestimmen.

Die US 2013/0096448 (Brooks) beschreibt eine kombinierte ECG-, ICG- und Phonoelektrode auf einem gemeinsamen Träger mit akustischer Kammer. Im Gegensatz dazu ist gemäß der vorliegenden Erfindung keine akustische Kammer vorgesehen.

Die Schriften US 8521264 und US 2010/0324404 beschreiben die Verwendung von maximal drei kombinierten ECG-ICG Elektroden, die alle am Thorax platziert sind.

Die US 6339722 (Heethaar) schlägt vor, den Thorax als ein Segment bei zwei Frequenzen und mit zwei verschiedenen Meßdistanzen zu vermessen, um Information über die Herztätigkeit zu gewinnen. In der US 6,560,481 (Heethaar) sind für die Impedanzmessung Elektrodenpositionen über der Clavikel und auf der linken Körperseite unter dem Sternum beschrieben. In der US 7904141 (Osypka) werden aus zwei Messungen der Impedanz oder aus der Applanationstonometrie LVET errechnet.

Die US 4807638 (Sramek) und die WO 89/03656 A1 schlagen vor, mit Hilfe von zwei Impedanzkardiogrammen über dem Herzen und in der Peripherie den Blutdruck zu errechnen.

Das Dokument EP 2319411 A2, auch veröffentlicht als WO 2007045006 A1, wird als nächstliegender Stand der Technik angesehen. Es offenbart ein Gerät und ein Verfahren zur elektrischen Messung von Körperfunktionen und Zuständen, das mittels am Körper befestigbarer Elektroden elektrische Messgrößen, die mit den Körperfunktionen und Zuständen korrespondieren, erfasst und aus den erfassten elektrischen Messgrößen die Körperfunktionen und Zustände errechnet, wobei zumindest in zwei seriell-geschalteten Körpersegmenten die Impedanzänderungen mit dem Herzschlag und/oder deren Ableitungen gemessen werden und aus den kombinierten Messgrößen ein Parameter für die Herzleistung errechnet wird.

Das Dokument EP 1275342 A2 beschreibt eine Vorrichtung zum Bestimmen des Herzzeitvolumens eines lebenden Subjekts. Die verbesserte Vorrichtung umfasst eine oder mehrere Elektrodenanordnungen oder Pflaster, die an der Haut des Subjekts in der Nähe der Brusthöhle befestigt sind. In einer Ausführungsform umfasst die Vorrichtung einen Konstantstromquellen-Impedanz-Kardiographie(ICG)-Monitor, der als eigenständiges System ausgelegt ist. In einer anderen Ausführungsform umfasst die Vorrichtung ein Modul, das zur Verwendung mit einem Host-Überwachungssystem angepasst ist, wobei letzteres EKG, Blutdruck und/oder andere Eingaben für das Modul bereitstellt.

Das Dokument WO 2004030535 A1 beschreibt ein Verfahren zur Messung des Volumens, der Zusammensetzung und Bewegung (HZV) elektrisch leitender Körperflüssigkeiten, beruhend auf der elektrischen Impedanz des Körpers oder eines Körpersegments, insbesondere für die Elektromechanocardiographie (ELMEC)- bzw. Impedanz-Kardiographie (IKG)-Messung zur Bestimmung haemodynamischer Parameter. Es wird ein Wechsel-Messstrom zumindest einer Frequenz in den Körper eingebracht und die Impedanz und deren Änderung über die Zeit des vom Wechsel-Messstrom durchflossenen, selben Körpersegmentes bei zumindest zwei unterschiedlichen Messlängen in Längsrichtung des Körpers gemessen.

Das Dokument US 2005/273015 A1 offenbart einen akustischen Sensor zur Erfassung von Herztönen.

Das Dokument US 2011/245688 A1 beschreibt einen Elektrokardiographen mit mehreren Sensoren, die jeweils eine Elektrode zur Erfassung vom Körper eines Patienten erzeugter elektrischer Impulse aufweisen. In einer Ausführungsform umfasst das System auch ein Accelerometer, das kommunikativ mit dem Elektrokardiographen gekoppelt ist, wobei das Accelerometer in der Lage ist, eine Bewegung des Körpers des Patienten zu erkennen und dem Elektrokardiographen Signale bereitzustellen, die die erkannte Bewegung anzeigen. Der Elektrokardiograph detektiert aus den empfangenen Bewegungssignalen eine bestimmte Art von Patientenbewegung und/oder Patientenposition und stellt eine Ausgabe basierend auf den Signalen, die die erfassten elektrischen Impulse anzeigen, und eine Ausgabe basierend auf den Signalen, die die erfassten Bewegungen anzeigen, bereit.

Bei den in den obigen Dokumenten beschriebenen Geräten ist die Messstrecke vollständig vom Messstrom durchflossen. Es findet zwar auch auf diesen Messstrecken Ionenleitung statt, diese wird zur Messung aber nicht benutzt, sondern nur der Messstrom und daraus abgeleitete Messgrößen, wie die Impedanz, gemessen.

Es besteht nach wie vor ein Bedürfnis nach einem EKG-Gerät mit erhöhtem Funktionsumfang bei gleichzeitig vereinfachter Bedienung.

Die vorliegende Erfindung löst diese Aufgabe durch Bereitstellen eines EKG-Geräts mit den Merkmalen des Anspruchs 1. Vorteilhafte Ausführungsformen der Erfindung sind in den Unteransprüchen dargelegt.

Das erfindungsgemäße EKG-Gerät ermöglicht eine besondere Vereinfachung der Vermessung, indem die beiden Elektroden an den Oberschenkeln wegfallen können und trotzdem der Körper ausschließlich mit den Elektroden der Extremitäten und der oberen Körperapertur in sechs Segmenten, nämlich in Thorax, Abdomen und jeweils 2 Beinen und Armen analysiert werden kann.

Im Gegensatz zu den erwähnten Veröffentlichungen baut die vorliegende Erfindung auf Basis neuer physiologischer Erkenntnisse auf der Verwendung eines herkömmlichen Mehrkanal-, -EKG-Geräts auf und ermöglicht es, mithilfe zumindest einer einzelnen Elektrode an der oberen Körperapertur komplexe Kreislauf- und Flüssigkeitsanalysen vorzunehmen. Die Erkenntnisse des Erfinders beinhalten u.a., dass die Verwendung einer herzfernen Impedanzkurve an einer Extremität zusätzlich zur Messung der Impedanzkurve am Thorax viel besser die Messung der Herzleistung und der Faserspannung des Herzens ermöglichen und damit auch für eine Abschätzung von biochemischen Parametern, wie z.B. einem Parameter des BNP und seiner Derivate, bestens geeignet sind. Weiters hat der Erfinder erkannt, dass die Messung der Impedanz bei zumindest zwei Frequenzen in sequentiell herznah und herzfern gelegenen Segmenten des Körpers einen exzellenten Parameter für ein Fluid Overload oder für Dehydrierung liefern und dass mit Hilfe der Zerlegung des Körpers in sechs Segmente, nämlich Arme, Beine, Thorax und Abdomen, auch das Gesamtkörper-TBW, ECW und ICW exzellent geschätzt werden kann und dass diese Parameter zur Messung der Herzleistung und biochemischer Herzparameter entscheidend beitragen. Als herznahes Segment wird der Thorax, als herzferne Segmente werden Abdomen und die Extremitäten bezeichnet. Wenn Thorax und Abdomen als gemeinsames Segment untersucht werden, werden diese als gemeinsames herznahes Segment betrachtet. Weiters zeigt der Erfinder, dass aus der Relation von aus der segmentalen Impedanz vorhergesagtem % Körperfett oder Fettmasse (FM) oder % Körperwasser = Total Body water (TBW), jeweils bezogen auf das Gesamtkörpergewicht, oder "lean body mass" (LBM) zur Ratio von extrazellulärem Wasser (ECW) zu intrazellulärem Wasser (ICW) also zur ECW/ICW Ratio oder auch zur ECW/TBW Ratio in einem Körpersegment oder im Ganzkörper erstmals auch eine Überhydrierung oder Unterhydrierung des Körpers erkannt werden kann. Weiters werden bei der Anwendung der vorliegenden Erfindung jene Teile des Körpers, die nicht von einem Wechselstromfeld durchströmt sind, als Ionenleiter verwendet, womit eine weitere Vereinfachung der Methode und der verwendeten Elektroden, speziell der aus zwei getrennten, jedoch elastisch verbundenen Branchen gebildeten Klemmelektroden, gegeben ist.

In der Folge werden als obere Thoraxapertur der Bereich des Halses, Nackens, Kopfes, Schultern und Arme, bevorzugt Oberarme sowie auch die Elektrodenpositionen V1, V2, als untere Thoraxapertur der Bereich des unteren Rippenbogens, Xiphoids, also die übliche Gegend zur Anbringung der Brustwandelektroden des EKGs, zB V4 bis V6, zB V4r bis V6r sowie die Grenze zwischen Brustwirbelsäule und Lendenwirbelsäule bezeichnet. Als unteres Rumpfende wird der Bereich des Beckens, die Nates und die proximalen Oberschenkel angesehen.

Die Erfindung wird nachfolgend anhand von Ausführungsbeispielen unter Bezugnahme auf die Zeichnungen näher ausgeführt.

Fig. 1 zeigt die Ableitung eines herkömmlichen Vielkanal-EKGs und eine Zusatzelektrode.

Fig. 2a bis 2l zeigen die jeweiligen Lokalisationen der Stromeinspeisung mit Wechselstromquelle, weiters sehr schematisch die sich so aufbauenden elektrischen Felder im menschlichen Körper.

Fig. 3a zeigt eine Ausführung einer Elektrode, um neben dem EKG auch die mechanische Tätigkeit des Herzens aufzeichnen zu können.

Fig. 3b zeigt eine weitere Ausführung einer Elektrode, um neben dem EKG auch die mechanische Tätigkeit des Herzens aufzeichnen zu können.

Fig. 4 zeigt eine Saugelektrode.

Fig. 5 zeigt eine weitere Ausführungsform einer Saugelektrode.

Fig. 6 zeigt eine weitere Ausführungsform einer Elektrodenkonfiguration.

Fig. 7 zeigt eine weitere Ausführungsform einer gemeinsamen Elektrode für die Aufzeichnung des EKG und für die Emission und Registrierung von zusätzlichen physikalischen Parametern.

Fig. 8 zeigt schematisch eine Universalelektrode mit Druckknopfverbindung - im Querschnitt.

Fig. 9 bis Fig. 13 zeigen Ergebnisse von Messungen an gesunden und kranken Menschen.

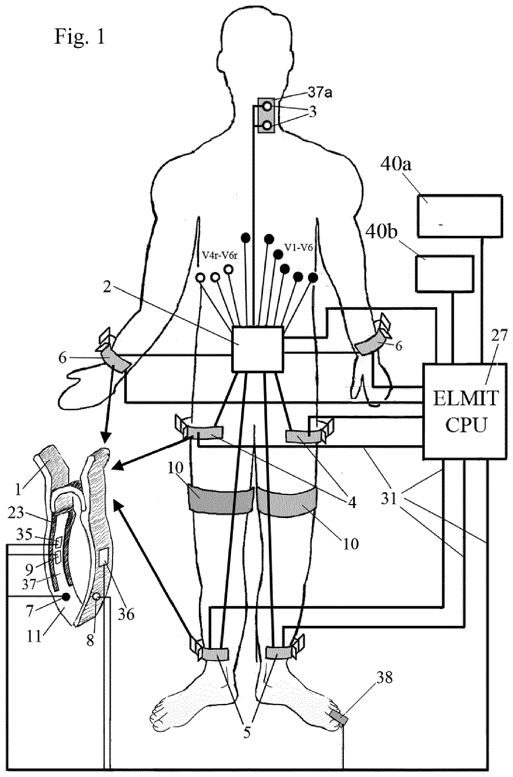

Fig. 1 zeigt die Ableitungen eines herkömmlichen Vielkanal-EKGs, bestehend aus den Extremitätenelektroden, hier z.B. als Doppelektrode -1- z.B. als Doppelklemmelektroden -1-ausgeführt, und andererseits, wie bekannt, aus den Brustwandableitungen ( V1-V6, eventuell ergänzt durch V4r-V6r), wobei zur Untersuchung des Flüssigkeitshaushaltes die einzelnen Elektroden mit Hilfe eines elektrischen oder elektronischen Schalters -2-, z.B. eines Multiplexers -2- zusammenschaltbar sein können bzw auch getrennt gemessen werden bzw. als Stromeinleitungselektroden und/oder Impedanzmesselektroden ausgeprägt werden können. Die möglichen Details der Armelektroden -6-, proximalen Beinelektroden -4- und distalen Beinelektroden -5- einer Körperhälfte sind dabei, wie in der Detailzeichnung in Fig. 1 links, wie mit den Pfeilen gekennzeichnet, ausgeführt. Mehrfache elektrische Leitungen -31-von den Elektroden sind allenthalben übersichtlichheitshalber in den Abbildungen nur als einzelne Leitungen gezeichnet. Durch nur eine zusätzliche Einfach- oder Doppel-Halselektrode -3- und durch eventuelle Zusammenschaltung z.B. von V4 bis V6 einerseits und V4r bis V6r andererseits, z.B. durch den Multiplexer -2-, kann mittels Impedanzmessung der Thorax hinsichtlich seiner Impedanz (z0) und des Flüssigkeitsgehaltes und der Änderung des Flüssigkeitsgehaltes mit dem Herzschlag (z.B. dZ, dZ/dt, dZ/dt max) genau untersucht werden. Wie ersichtlich sind die Elektroden z.B. nicht wie bei Impedanzmessungen üblich, an den Händen und Füßen, sondern am distalen Unterarm sowie distalen Unterschenkel angebracht. Doppelektroden sind besonders für die Vierpunktmessung der Impedanz mit außen liegenden Einspeiselektroden und innen liegenden Meßelektroden günstig. Die Unterbringung auf einem gemeinsamen Träger -37-, -37a- erleichtert die Handhabung, ist aber nicht unbedingt erforderlich. Die Stromeinspeisung kann dafür z.B. zwischen der oberen Thoraxapertur z.B. einer Halselektrode (auch der Nacken bzw. der Kopf bzw. die Schultern käme für die Platzierung der Halselektrode in Frage) bzw. auch der/den Armelektrode/n -6 - einerseits und der/den proximalen Beinelektrode/n -4- und/oder distalen Beinelektrode/n -5-andererseits erfolgen, Durch die Messstrecke zwischen der Doppel-Halselektrode -3-einerseits und z.B. V4 bis V6 andererseits kann die elektrische Impedanz bzw. der Wirkwiderstand und der Blindwiderstand des linken Thorax untersucht werden, durch Zusammenschalten von z.B. V 4 r bis V6 r, kann eine Messstrecke zwischen der Halselektrode -3- und der rechten Thoraxhälfte erreicht werden.

So können Flüssigkeitsansammlungen in der linken Thoraxhälfte bzw. in der rechten Thoraxhälfte, z.B. durch Pleuraerguss, oder Flüssigkeitsansammlung im Bereich der Lunge, z.B. durch Pneumonie, Atelektase oder Lungenödem bzw. auch die durch den Herzschlag verursachte Änderung der Impedanz erkannt werden. Auch kann jede der Brustwandelektroden allein zur Messung der Impedanz zwischen der Halselektrode -3-einerseits und einer der Brustwandelektroden andererseits herangezogen werden, nur ist dann die Reproduzierbarkeit der Messung nicht mehr so gut gegeben. Dies wird wettgemacht, wenn die Elektroden z.B. V4 , V5, V6 einzeln zur Messung verwendet werden und dann eine eventuelle Mittelwertbildung der Resultate erfolgt. Somit kann auch ohne Zusammenschalten mathematisch die Impedanz des gesamten Thorax errechnet werden. Alternativ könnte auch eine oder mehrere der Ableitungen V1-V3 statt der Halselektrode zur Messung der Impedanz zwischen diesen Ableitungen und den Ableitungen V4 bis V6 herangezogen werden, jedoch ist dann die Messstrecke sehr kurz und das Signal-Rauschverhältnis nicht so günstig.

Das Einleiten des Stromes für die Impedanzmessung erfolgt wie gesagt in diesem Fall vorteilhaft über eine oder beide Armelektroden -6- und/oder über die Halselektrode -3-einerseits und entweder über die eine oder beide proximalen Beinelektroden -4-, und/oder aber auch über die eine oder zwei distalen Beinelektroden -5- (entweder Saug-, Klebe- Band- oder Klemmelektroden) am linken und rechten Bein andererseits, am Ort, wo sie auch für die EKG-Ableitungen verwendet werden. Für die separate Untersuchung der Flüssigkeit bzw. der Flüssigkeitsverschiebung in den beiden Beinen können die Beine durch Zusammenschalten oder Trennen im Multiplexer -2- separat oder gemeinsam untersucht werden bzw. auch hier ohne Zusammenschalten die Gesamtimpedanz von linkem und rechtem Bein errechnet werden. Auch die Armelektroden -6- sind vorteilhaft z.B. als Doppelektroden -1-, , im speziellen Fall als Doppelklemmelektroden -1- ausgeführt. Die elektrisch leitenden Elektroden -23- müssen jedoch nicht an derselben Branche der Doppelklemmelektrode -1-, wie in Fig. 1 gezeigt sitzen sondern die eine der beiden elektrisch leitenden Elektroden -23- könnte auch auf der gegenüberliegenden Branche der Doppelklemmelektrode -1- angebracht sein.

Die zusätzliche Messung der Impedanzänderung mit dem Herzschlag in den Extremitäten, Armen oder Beinen ermöglicht auch eine sehr gute Beurteilung der Kraft des Herzschlages, besonders wenn dies gemeinsam mit der Impedanzänderung am Thorax verwendet wird.

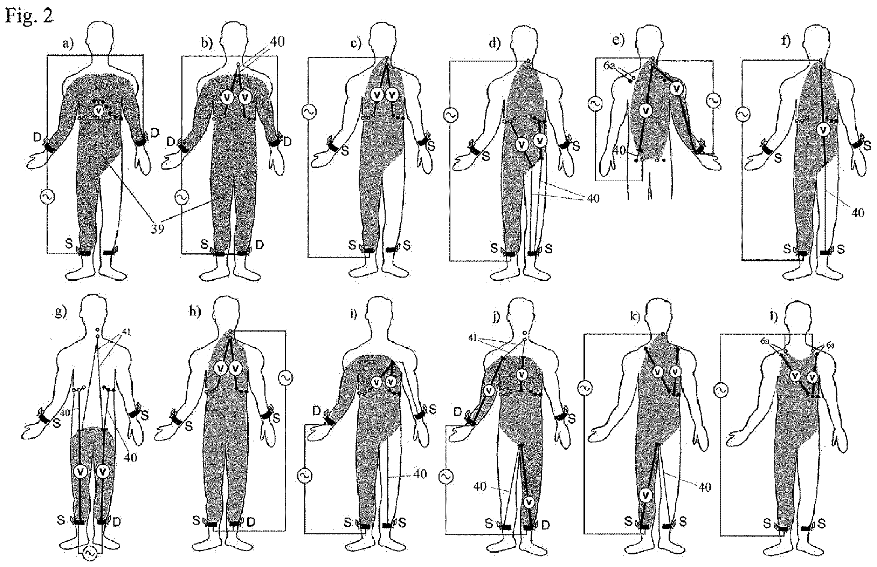

Auch der Flüssigkeitshaushalt des Patienten kann mit obiger Anordnung exzellent erkannt werden, da der Körper durch die zusätzliche Halselektrode -3-, die Armelektroden -6- und durch die proximalen Beinelektroden -4- und die distalen Beinelektroden -5-, sowie die Brustwandelektroden V3-V6 und/oder V3r-V6r, in sechs Abschnitte, nämlich die zwei Arme, den Thorax und das Abdomen bzw. die zwei Beine unterteilt ist. Auch die Beschaltung der Elektroden, wie in Fig. 2 später angeführt, erlaubt die Unterteilung in die sechs Abschnitte, auch ohne Verwendung der proximalen Beinelektroden -4-. Die Trennung von Abdomensegment und Thoraxsegment für die Körperanalyse ist deswegen so günstig, weil diese auf Grund der anatomischen Unterschiede (z.B. die luftgefüllten Lungenbläschen) unterschiedlich auch in die Berechnung der Körperzusammensetzung eingehen müssen. In jedem dieser einzelnen Segmente wird bei mehreren Frequenzen, eventuell auch einem kompletten Frequenzsweep der Wechselstromwiderstand (Impedanz) bzw. der Wirkwiderstand (Resistanz) und der Blindwiderstand (Reaktanz) der einzelnen Körperabschnitte analysiert und daraus das ECW, das TBW, damit auch das ICW, vor allem auch das Verhältnis ECW/TBW oder ECW/ ICW analysiert. Besonders bewährt sich die Analyse der Segmente bei zumindest zwei Frequenzen z.B. einerseits zwischen 1 Hz und 10 Hz, z.B 5 Hz, und andererseits bei höheren Frequenzen als 100 Hz, z.B. auch 400 bzw. 800 Hz, oder auch durch einen Frequenzsweep, weil so das Verhältnis von extra- zu intrazellulärer Flüssigkeit ermittelt werden kann, welches unabhängig von den Dimensionen des untersuchten Segmentes ist. Für die Messung der Impedanzänderung mit dem Herzschlag (dZ, dZ/dtmax) wird üblicherweise eine Frequenz von ca. 40 kHz verwendet. Auch diese oder eine ähnliche Frequenz sollte vom hämodynamischen EKG Gerät geliefert werden. Die Stromeinspeisung für die einzelnen Segmente erfolgt z.B. jeweils durch außerhalb des untersuchten Segmentes liegende Elektroden, z..B. für die Vermessung des Thorax, zwischen der äußeren der Doppel-Halselektrode -3- bzw. Kopfelektrode bzw. Armelektroden/n -6- einerseits und einer der distal von den Brustwandelektroden liegenden Elektroden, z.B. den proximalen -4- oder distalen Beinelektroden -5- andererseits, die Vermessung des Abdomensegmentes durch Einspeisung an den Armelektroden -6- und/oder Halselektroden - 3- einerseits und den distalen Beinelektroden -5- andererseits, die Vermessung der Arme, durch Einspeisung an den distalen Doppelarmelektroden -6- und der Halselektrode -3-. Das gemessene Thoraxsegment liegt zwischen der proximalen Halselektrode einerseits und einer oder mehreren Brustwandelektroden V4 bis V6 bzw. V4r bis V6r andererseits. Für die Vermessung der Impedanzänderung mit dem Herzschlag am Thorax können auch die proximalen Beinelektroden -4- herangezogen werden, weil das Abdomen nicht zum dZ/dt beiträgt. Es ist auch an weitere Vereinfachungen, wie z.B. die Ausführung der proximalen Beinelektrode -4- als Einfachelektrode gedacht. Selbstverständlich ist auch an den üblichen Defibrillationsschutz gedacht. Da das Messmodul nahe einer zentralen Stange, an dem der Auslegerarm mit den Patientenkabeln montiert ist, liegen soll, sollten die Patientenkabeln entlang des Auslegerarms laufen und am Ende desselben frei für den Gebrauch hängen. Damit sich diese nicht verwirren, wird vorgeschlagen einen Abstandhalter am Ende des Auslegerarms vorzusehen, der die Patientenkabel je nach ihrer Position am Körper räumlich separiert.

Es sind auch weitere Vereinfachungen vorgesehen, z.B. eine besondere Vereinfachung der Vermessung, wobei dann die beiden Elektroden an den Oberschenkeln wegfallen können und trotzdem der Körper ausschließlich mit den Elektroden der Extremitäten und der Brustwand in 6 Segmenten, nämlich in Thorax, Abdomen und jeweils 2 Beinen und Armen analysiert werden kann. In vielen Fällen genügen dann an den Messstellen Einfachelektroden, für die z.B. die EKG-Elektroden herangezogen werden können, wenn nämlich die Einspeisung des Stromes anderswo erfolgt.

In Fig. 2a bis 2l sind die jeweiligen Lokalisationen der Stromeinspeisung mit Wechselstromquelle gezeichnet, weiters sehr schematisch die sich so aufbauenden elektrischen Wechselstromfelder -39- als punktierte Flächen. Die Messstrecken sind ebenfalls mit den üblichen Symbolen, hier z.B. mit V, gekennzeichnet. In den jeweiligen Teilfiguren a-j ist dabei gekennzeichnet, ob es sich als Minimalerfordernis in der jeweiligen Abbildung um eine Doppelelektrode, symbolisiert mit D, oder um eine Single -Elektrode oder symbolisiert mit S handelt. Teile des menschlichen Körpers, in denen kein Stromfeld aufgebaut ist, werden dabei nur als elektrischer Leiter -40- , genauer gesagt Jonenleiter -40- benutzt, da ja der Elektrolytgehalt des Körpers mit seiner hohen extrazellulären und intrazellulären Ionenkonzentration einen guten Leiter, speziell für den Wechselstrom darstellt. Dabei kann es sich bewähren, auch für die Messung der Impedanz und deren Änderung mit dem Herzschlag höhere als die oft gebräuchlichen 40 kHz zu verwenden, um eine Leitung des Wechselstromes auch durch das Intrazellulärwasser sicher zu gewährleisten. Fig. 2 zeigt weitere mögliche Stromeinspeislokalisationen und Lokalisationen für den Abgriff der Impedanz, möglichst unter bevorzugter Verwendung der EKG-Elektroden. So kann z.B. die Erdungselektrode (üblicherweise als schwarze Elektrode am rechten Bein) einerseits und andererseits einer der beiden Arme oder beide Arme für die Stromeinspeisung herangezogen werden (Fig. 2a). Die Erdungselektrode kann dazu problemlos herangezogen werden, wogegen sich bei den Armen eine Doppelektrode bewährt, da die EKG- Ableitelektroden an den Armen nicht für die Einspeisung des Stromes verwendet werden sollten (Fig. 2a). Zur Messung der Impedanz bzw. deren Teilkomponenten Resistanz und Reaktanz können die EKG-Elektroden hingegen herangezogen werden. Hier wird immer nur von Impedanz gesprochen, auch wenn die Teilkomponenten und die Änderung der Impedanz mit dem Herzschlag (dZ, dZ/dt max und weitere Characteristika) damit gemeint sein können. So kann z.B. die Impedanz zwischen der Halselektrode -3- und den Elektroden, wie sie nicht für die Zusammenschaltung als zentrales Terminal nach Wilson, nämlich eine der Elektroden V1 bis V6, z.B. V4 bis V6 für die Messung der Impedanz und/oder deren Änderung mit dem Herzschlag herangezogen werden, wobei hier beispielsweise die Einspeisung über beide Arme und beide Beine erfolgt (Fig. 2b), wobei auch ein Bein und ein Arm genügen könnte. Es kann auch die Impedanz zwischen den einzelnen Brustwandelektroden, das sind die, die nicht für das zentrale Terminal Wilson zusammengeschaltet werden können, gemessen werden. Z.B. könnte das sein zwischen V1 und V6 (ev. auch V4 oder V5) oder auch zwischen V1,V2 optional zusammengeschalten einerseits und V6,V6r (ev. auch V4 oder V5 mit V4r und V5r) optional zusammengeschalten andererseits (Fig. 2a). In diesem Fall würden die Elektroden V1, V2 der oberen Thoraxapertur zugeordnet werden. Die rechtsventrikulären Ableitungen sind nur eine zusätzliche Option, auch ohne diese kann eine komplette Analyse der Herztätigkeit und der Flüssigkeitsverteilung im Körper durchgeführt werden. Die rechtsventrikulären Ableitungen haben den Vorteil, dass der linke mit dem rechten Thorax bzgl. Impedanz verglichen werden kann und dass gleichzeitig die Diagnose eines Hinterwandinfarktes im EKG erleichtert wird. Auch zusätzliche Ableitungen wie Nehb Ableitungen oder Franksche Ableitungen sind möglich.

Dies sind nur Beispiele, auch das Zusammmenschalten von anderen Elektroden, wie sie nicht im zentralen Terminal Wilson zusammengeschalten werden, ist vorgesehen. Bei Verwendung nur einer einzelnen weiteren Elektrode, die zwischen zwei der für das zentrale Terminal Wilson vorgesehenen Elektroden liegt, nämlich zwischen linker und rechter Armelektrode -6-, Einthoven Ableitung I (den Elektroden Rot und Gelb des konventionellen EKG), könnte die Impedanz und deren Änderung mit dem Herzschlag besonders genau gemessen werden, da die Messstrecke länger und damit auch das Signalrauschverhältnis besser wird. Diese Elektrode würde am Hals -3-, Nacken, oberer Thoraxapertur oder am Schädel zu liegen kommen. Durch eventuelle Verwendung einer Doppelektrode -3- an dieser Lokalisation kann nicht nur der Strom eingespeist sondern auch die Impedanz abgegriffen werden (Fig. 2c). Für die Vermessung des Thoraxsegmentes könnte die Einspeisung einerseits an der äußeren Halselektrode und/oder an den Armelektroden -6- und andererseits wenn vorhanden, an den proximalen Beinelektroden -4- oder distalen Beinelektroden -5- erfolgen, die Spannungsmessung zwischen innerer Halselektrode und Brustwandelektroden, wobei der linke und rechte Thorax im gesamten durch Zusammenschalten z.B. von V5;V6, V5r und V6r und/oder auch nur rechts und links z.B. über V4-V6 einerseits oder andererseits über V4r-V6r vermessen werden sollten (Fig. 2c). Für die Messung des Thoraxsegmentes ist es nicht wesentlich ob der Strom nur über die Erdungselektrode (schwarz, am rechten Bein) oder über beide Beine nämlich die Erdungselektrode und diejenige der 3 Elektroden, die für das zentrale Terminal Wilson zusammengeschaltet werden und die nicht für die Ableitung I nach Einthoven herangezogen wird, eingespeist wird. Dies deswegen, weil auch bei Einspeisung nur über ein Bein das Stromfeld im Rumpf bereits äußerst homogen ist (Fig. 2c). Die Vermessung des Thorax könnte auch durch Einspeisung an einem Arm einerseits und einem oder beiden Beinen andererseits erfolgen, die Messung der Spannung zwischen dem jeweils kontralateralem Arm und den Brustwandelektroden (Fig. 2i) bzw. falls vorhanden, an der oder den proximalen Beinelektroden -4- andererseits. Der kontralaterale Arm in Fig. 2i würde in diesem Fall nur als Stromleiter fungieren. Die Vermessung des dz/dt am Thorax ist hingegen nicht günstig zwischen Halselektrode -3- und der/den den distalen Beinelektroden -5-, da dann nicht zwischen der Volumsänderung mit dem Herzschlag am Thorax und der in den Extremitäten unterschieden werden kann. Die Messung der Volumsänderung, z.B. dZ/dtmax am Thorax und an einer Extremität ist jedoch für die Beurteilung der Herzleistung besonders günstig, speziell wenn zusätzlich noch das Verhältnis von extra- zu intrazellulärer Flüssigkeit berücksichtigt wird. Aus diesen drei kombinierten Parametern läßt sich besonders gut eine Einschränkung oder Verbesserung der Herzleistung erkennen.

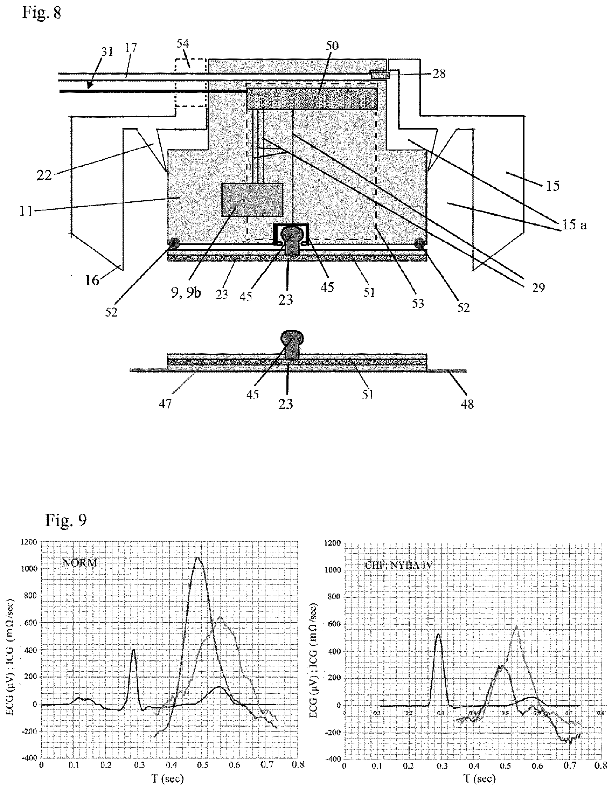

Für die Vermessung des Abdomens ist z.B. die Stromeinspeisung zwischen einer der beiden oder beiden Armelektroden -6- einerseits ( alternativ der/den Halselektroden einerseits), und einer der distalen Beinelektroden -5- andererseits, die Impedanzmessung zwischen z.B. einer oder mehreren zusammengeschalteten Brustwandelektroden einerseits und falls vorhanden, der jeweils kontralateralen proximalen Beinelektrode -4- bzw. distalen Beinelektrode -5-, die nicht für die Stromeinspeisung vorgesehen ist, andererseits möglich.(z.B. Fig. 2d). Für die Vermessung des Abdomens erfolgt die Stromeinspeisung z.B. zwischen einem der Beine und einem oder beiden Armen und die Spannungsmessung zwischen jeweils dem kontralateralen Bein und den Brustwandelektroden. Für die Vermessung eines Beinsegmentes erfolgt z.B. die Stromeinspeisung zwischen jeweils den äußeren der beiden distalen Beinelektroden -5- und die Impedanzmessung zwischen jeweils der inneren distalen Beinelektrode -5- des untersuchten Bein einerseits und den Brustwand- und/oder Hals- und/oder Armelektroden -6-andererseits (z.B. Fig. 2g). So kann auch weiters für die Messung der Arme der Strom zwischen der äußeren Armelektode -6- und der äußeren Halselektrode -3- eingespeist und die Spannung zwischen der inneren Halselektrode und der inneren Armelektrode -6- abgeleitet werden (Fig. 2e). Für die Vermessung des linken Armes gilt dies spiegelbildlich und in analoger Weise, wobei naturgemäß auch andere Einspeispunkte möglich sind. Eine weitere Vermessung des Armes und des Thorax ist in Fig. 2j dargestellt. Eine noch weitere Vereinfachung ist durch einen möglichen Wegfall auch der Halselektrode gegeben: Für die Vermessung der Armsegmente müßte dazu zwischen beiden Armen eingespeist, und für die Analyse des interessierenden Armes zwischen diesem einerseits und andererseits an den Brustwandelektroden V4 bis V6 bzw. V4r bis V6r und/oder proximalen -4- oder distalen Beinelektrode/n -5- die Spannung gemessen werden. Es ist offensichtlich, dass eine Kombination aus den verschiedenen Abbildungen 2a bis 2l möglich ist. Eine besonders praktikable und bevorzugte Ausprägung gelingt aus einer Kombination der Einspeisungen aus Fig. 2g und 2h, wenn auf proximale Beinelektroden -4- verzichtet werden soll. Dabei kann alternativ die Halselektrode oder eine oder mehrere Brustwandelektroden für die Vermessung der Beine verwendet werden, wie das in Fig. 2g gezeigt ist. Auch die Arme könnten in der Fig. 2g gemeinsam mit den Beinen für die Impedanzmessung der Beine herangezogen werden (nicht gezeigt). Andererseits wäre es auch möglich, zwischen einer distalen Beinelektrode -5- und einer der in der oberen Körperhälfte liegenden Elektroden den Wechselstrom einzuspeisen und zwischen den distalen Beinelektroden -5- die Impedanz zu messen, wobei dann nur ein Bein vom Wechselstrom durchströmt und das andere Bein als elektrischer Ionenleiter -40- benützt wäre (Fig. 2 j und Fig 2 k). So gelingt es ausschließlich mit Hilfe der Extremitätenelektroden und der Brustwandelektroden mit oder ohne Halselektrode, jedes einzelne der sechs Segmente zu analysieren. Die Präzision der Messungen wird allerdings durch die zusätzliche Verwendung der Halselektroden -3- und zusätzlicher proximaler Beinelektroden -4- weiter verbessert. Für die Erstuntersuchung ist besonders das komplette EKG mit Brustwandelektroden und Extremitätenelekroden interessant, bei Fehlen von Herzbeschwerden wird man in der Folge eventuell mit der Information mit den Extremitätenableitungen auskommen. So kann bei Einspeisung des Stromes zwischen der Elektrode an der oberen Körperapertur (z.B. Halselektrode -3- , Schulterelektroden -6a-) und einer distalen Beinelektrode und Messung der Impedanz zwischen Halselektrode und distaler Elektrode am anderen Bein das dz und dz/dt des herznahen Segmentes gemessen werden (Fig. 2f) . Bei Stromeinspeisung zwischen den beiden distalen Beinelektroden und Messung der Impedanz zwischen der Elektrode an der oberen Thoraxapertur oder einer Brustwandelektrode einerseits und einer distalen Beinelektrode andererseits wird das dZ und dZ/dT am herzfernen Körpersegment gemessen (Fig 2g). Wenn die Extremitätenelektroden für die Ableitung I nach Einthoven zwischen rechtem und linkem Arm als Schulterelektroden -6a- ausgeführt sind, können diese Elektroden auch die Funktion der Impedanzmessung an der oberen Thoraxapertur übernehmen und die Halselektrode -3- kann nur Einzelektrode zur Stromeinspeisung ausgeführt sein bzw. die distalen Armelektroden können naturgemäß entfallen (Fig. 2k), wobei dann eine Analyse der Arme nicht mehr möglich ist, wobei diese nur ca 7% des Körpervolumens ausmachen. Speziell auch für die Ergometerbelastung ist diese Anordnung besonders sinnvoll. Fig 2 1 zeigt eine Platzierung von Doppelektroden (z.B. Klebe- oder Saugelektroden) an der oberen Thoraxapertur, nämlich an beiden Schultern über die einerseits die EKG Schreibung der Extremitätenableitungen erfolgen kann andererseits kann an diesen Stellen auch der Strom für die Impedanzmessungen eingespeist werden. Dies wäre eine der Versionen mit geringen Änderungen gegenüber dem herkömmlichen EKG. Eine Kombination der verschiedenen Variationen aus Fig 2a bis 2l ist natürlich vorgesehen. Es muß hier noch betont werden, daß die Ergebnisse der Impedanzmessungen kritisch von den Positionen der Stromeinspeisung und Spannungsmessung abhängen, weswegen Doppelektroden auf einem gemeinsamen Träger von Vorteil sind. Es muß auch nochmals betont werden, dass es von Vorteil ist, die Elektroden z.B. die Brustwandelektroden nicht zusammenzuschalten, sondern z.B. die Impedanz und deren Änderung mit dem Herzschlag vom Hals nach V4, V5 und V6 jeweils separat auch wenn in Fig. 8 der Klarheit halber immer nur ein Teil dieser Messsstrecken eingezeichnet ist, zu messen, und dann durch Mittelwertbildung der Messungen noch genauere Ergebnisse zu erhalten. Mit bis zu 4 Doppelektroden oder auch Einfachelektroden am Rücken wie in Fig. 2e gezeichnet können die Extremitätenableitungen (statt der Arm und Beinelektroden) speziell bei der Ergometrie verwirklicht werden, und gleichzeitig die Änderung der Thoraximpedanz mit dem Herzschlag (dZ bzw dZ/dtmax) um so die Änderung der Herzbeschleunigung unter der Ergometrie, wie sie durch in erster Linie durch Adrenalin verursacht wird. Damit kann die Sensitivität der üblichen Ergometrie weiter erhöht werden, wenn nämlich ein Anstieg des dZ oder dZ/dtmax schwächer ausgeprägt ist als beim Gesunden. Die Brustwandelektroden, die bei der Ergometrie weiterhin anzulegen sind, sind natürlich in dieser Rückenansicht Fig. 2e nicht sichtbar. Minimal genügt bei Verwendung einer Einspeiselektrode am Hals eine Doppelektrode am unteren Ende des Rückens, falls die Halselektrode zur Einspeisung fehlt, kann dazu auch eine oder zwei Doppelektroden an der oberen Thoraxapertur am Rücken verwendet werden. So sind Arme und Beine frei für die Bewegung. Die Vermessung eines Armes ist in Fig. 2e auch gezeichnet, wobei dies nicht gleichzeitig mit der Rückenvermessung stattfinden wird.

In den beschriebenen Abbildungen wird z.B. das Prinzip der Vierpunktmessung mit außen liegenden Strom- und innen liegenden Spannungselektroden verwirklicht, wobei auch eine Zweipunktmessung angedacht ist. Es soll auch verständlich gemacht werden, dass die angeführten Beispiele weiter modifiziert werden können und dass immer dann, wenn die Ableitung der Impedanz entfernt von der Einprägung des Stromes erfolgt, verständlicherweise an den jeweiligen Einprägungs- oder Ableitpositionen Einfachelektroden genügen. Auch ein Zusammenlegen von verschiedenen Einspeisorten z.B. Hals und Arme würde die Stromfelder z.B. am Thorax noch homogener machen. Es ist auch offensichtlich, dass die Stromfelder nicht so homogen wie gezeichnet sein können.

Wie in Fig. 1 , Fig. 3 bis Fig. 8 gezeigt, ist es möglich, an allen Elektrodenkörpern -11- auch zusätzliche Sensoren, wie z.B. Temperatursensoren -35- oder auch Lichtquellen -7- z.B. LEDs und Lichtsensoren -8- am Elektrodenkörper -11- der Klemmelektroden -1-, der elastischen Saugglocken -15- oder den Klebeelektroden -46- unterzubringen. Die LEDs -7-könnten z.B. gegenüber von den Lichtsensoren -8- z.B. auf den gegenüberliegenden Branchen der Klemmelektrode (Fig. 1) angebracht sein. auch könnten die Lichtquellen -7- und die Lichtsensoren -8- nebeneinander liegen und die Reflexion des Lichtes durch das arterialisierte Blut und dessen Pulsation gemessen werden. Auch hier könnte es sich einerseits um Saugelektroden handeln, andererseits könnten auch in bekannter Weise Klemmelektroden oder elastische Gurte zur Fixierung der Elektroden dazu verwendet werden. Dies betrifft die Elektroden I, II und III, wie sie zu dem zentralen Terminal Wilson zusammengeschaltet werden können, bzw. die Erdungselektrode. Auch hier können sich mechanische Aufnehmer, -9- z.B. Accelerometer -9b-, bewähren, um z B die Stosswelle des Blutes, die Durchblutung und die Volumsänderung mit dem Herzschlag auch außerhalb des Thorax, so auch die Pulswelle gleichzeitig mt Aufzeichnung der Änderung des z0, dZ und dZ/dt max zu registrieren. Damit können z.B. Durchblutungsstörungen einfach erkannt, bzw. auch die Pulswellengeschwindigkeit zwischen den einzelnen Elektroden gemessen werden. Wenn die Masse des Elektrodenkörpers -11- klein ist, wie z.B. bei den Brustwandelektroden, kann das Accelerometer z.B. in fixer Verbindung mit dem Elektrodenkörper -11- stehen, ist der Elektrodenkörper-11- gross, wie z.B. bei den Klemmelektroden -1- kann es vorteilhaft sein, den mechanischen Aufnehmer -9- beweglich, z.B. über eine Membran -13- an der Elektrode zu lagern. Dieses Problem ergibt sich verständlicherweise an Klebeelektroden -46- , z.B. an einer Spotklebeelektrode -46- oder an einer Bandklebeelektrode oder an einer mit einem elastischen Band nieder gehaltenen Elektrode nicht, da die Masse des Elektrodenkörpers -11-von vornherein klein ist. Mit Temperatursensoren -35- könnten z.B. Temperaturunterschiede zwischen den verschiedenen Elektrodenpositionen, wie sie z.B. duch Durchblutungsstörungen entstehen können, automatisch erfaßt werden. Mit Positionssensoren -36-, z.B. auf elektromagnetischer Basis bzw. Laufzeitmessung, bzw. Radiointerferometrie, bzw. Triangulation könnte so auch die Distanz zwischen den einzelnen Sensoren oder deren Position im Raum erkannt werden. Die Distanz wäre z.B. von der Halselektrode -3- oder den Brustwandelektroden V1-V6 oder V1-V6r zur proximalen Beinelektrode -4- bzw. distalen Beinelektrode -5- interessant, umso automatisch die Pulswellenlaufzeit der mechanischen Aufnehmer -9- oder Volumswellenlaufzeit mit Hilfe der Impedanzmessung oder Durchblutungslaufzeit mit Hilfe der Lichtsensoren -8- zu entdecken bzw. hilft auch die Distanzmessung um die Impedanzwerte auf die Länge des Segmentes zu normieren.

Durch zusätzlich angebrachte aufpumpbare Manschetten -10-, in denen der Druck verändert werden kann, können so auch besonders Verengungen oder Verschlüsse der Blutbahn erkannt werden, bei dem z.B. bei einer vorliegenden Verengung der Blutbahn, die Pulswelle oder die Volumswelle, erst bei niederen Manschettendrücken erscheint, als im gesunden Zustand. Alle diese Registrierungen erfolgen während der Durchführung eines normalen EKGs, auch innerhalb derselben Zeit, so könnte z.B. während der Aufzeichnung des Rhythmusstreifens eine zusätzliche grosse Informationsmenge über die mechanische Herztätigkeit bzw. die Funktion der Gefäße gewonnen werden. Alle angeführten Elektroden könnten als Klebeelektroden -46-, Saugelektroden oder Klemmelektroden ausgeführt sein bzw. durch ein elastisches Band am Körper fixiert werden. So ist z.B. die Halselektrode in Fig. 1 als Doppelspotelektrode -3- auf einem gemeinsamen Träger -37-, z.B. einer gemeinsamen Klebefolie -37a- ausgeführt. Wenn die Halselektrode -3- über der Vena jugularis angebracht ist, kann auch eine Venenpulskurve aufgezeichnet werden, was sich z.B. bei Herzinsuffizienz und Pericarditis bewährt. Auch mehrere zusammengeschaltete Spotelektroden wären links und rechts am Hals leicht anzubringen. Sollten mehrere Sensoren an den Elektroden angebracht sein, würden eventuell auch mehrere elektrische Zuleitungen ev. auch geschirmte Zuleitungen, zu den Elektroden erforderlich werden, was die Beweglichkeit der Kabel einschränken könnte. Um dies zu verhindern, könnten die gewonnenen Signale auch über Funk an ELMIT -27- und die CPU -27- zur weiteren Auswertung übertragen werden. Auch ist daran gedacht, dass zusätzliche Sensoren auch außerhalb der EKG-Elektroden angebracht werden können. So ist daran gedacht, einen Lichtemittter und Lichtsensor z.B. in Form eines Pulsoximeters -38- an zumindest einer der Akren anzubringen und damit die Sauerstoffsättigung und die Pulswelle, z.B. auch die Form der Pulswelle zu analysieren. In Kombination mit der zumindest einen aufpumpbaren Manschette -10- platziert am Arm, Oberschenkel oder Unterschenkel, kann damit auch die Durchblutung bzw. deren Störung in den verschiedenen Extremitäten analysiert werden, z.B. auch in bekannter Weise in Form des Ankle-Brachial Index.

Eine weitere Verbesserung des herkömmlichen EKGs wird dadurch erreicht, dass auch eine oder mehrere Elektroden an der Brustwand, das sind die Elektroden, die nicht für das zentrale Terminal Wilson zusammengeschaltet werden, auch für die Aufzeichnung eines Phonokardiogramms bzw. von mechanischen Erregungen geeignet sind. Für ein optimales Signal bewährt sich eine ca. 30 Grad Linksseitenlage, was z.B. durch einen Keilpolster erleichtert werden kann.

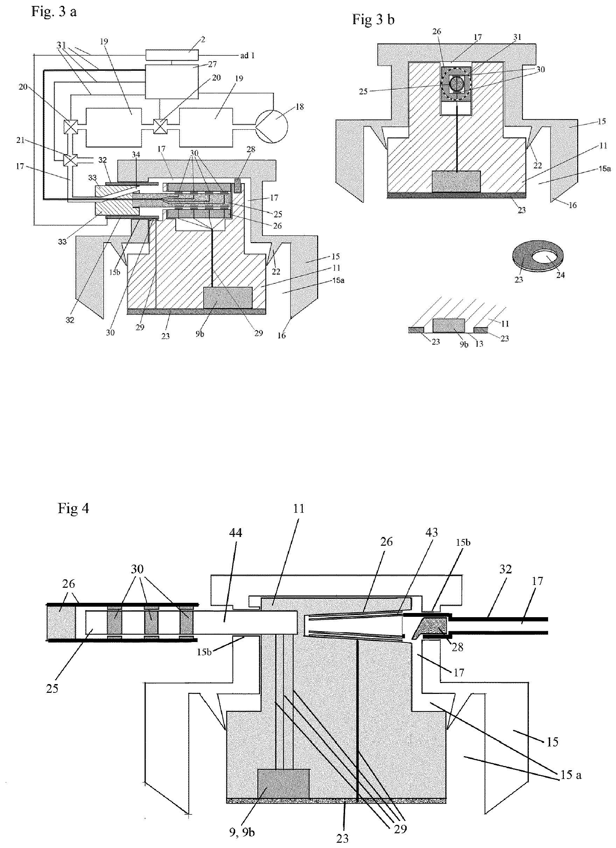

In Fig. 3 wird dazu gezeigt, wie diese Elektroden ausgeführt sein müssen, um neben dem EKG auch die mechanische Tätigkeit des Herzens aufzuzeichnen: Mit -11- ist dabei ein Elektrodenkörper -11-, z.B. annähernd kreisförmig oder oval beschrieben, in dessem Inneren z.B. symmetrisch oder asymmetrisch ein mechanischer Aufnehmer -9- z.B. Beschleunigungssensor -9b- untergebracht ist. Bei diesem mechanischen Aufnehmer -9-könnte es sich z.B. um ein Accelerometer -9b-, handeln, welches zur Gänze von der durchgehenden, elektrisch leitenden Elektrode -23- abgedeckt sein könnte, wie dies im oberen Teil und unteren Teil der Fig. 3 gezeigt ist. Der mechanische Aufnehmer -9- z.B. Accelerometer -9b- könnte auch andererseits an einer flexiblen, möglichst wenig dämpfenden Membran -13-, z.B. innerhalb der Öffnung -24- der elektrisch leitenden Elektrode -23-angebracht sein, um keine Dämpfung durch die Masse des Elektrodenkörpers -11- zu erfahren, wie dies in der rechten Mitte der Fig.3 gezeigt wird. Dieser Accelerometer -9b-kann die Pulsation des Herzens, wie z.B. den Herzspitzenstoß, das Apexogramm, oder auch die vom Herzen ausgehenden Schallschwingungen, wie z.B. die Herztöne und Herzgeräusche registrieren. Sollten mehrere mechanische Aufnehmer -9- innerhalb der verschiedenen Brustwandelektroden V1 bis V6 angebracht sein, können auch Verzögerungen der Bewegung des Herzens von einem Herzabschnitt zum anderen aufgezeichnet werden können, wie sie z.B. durch nicht funktionsfähige Areale des Herzens; z.B. nach Herzinfarkt, entstehen können. Um den mechanischen Aufnehmer -9-, z B das Accelerometer -9b- für Phonocardiogramm und für die Registrierung der Bewegung des Herzens verwenden zu können, sollte für die Verarbeitung des Signals zumindest ein elektronisches Filter vorhanden sein. Für die Aufzeichnung des Phonocardiogramms ist dafür ein Hochbassfilter günstig, welches Frequenzen zwischen ca. 50 bis 70 Hz, bevorzugt ca. 70 Hz einerseits und ca. 1000 Hz andererseits durchläßt, für die Verwendung z.B. im Apexogramm ein Tiefpassfilter, welches Frequenzen zwischen ca. 0,1 einerseits und ca. 20 bis 70 Hz, bevorzugt ca. 30 Hz andererseits durchläßt. Besonders vorteilhaft ist die parallele Ausführung von zumindest zwei elektronischen Messstrecken sodass beide Frequenzanteile parallel und ev. unterschiedlich nachbearbeitet werden können. So können aus dem Apex Cardiogramm und dem Phonocardiogramm gleichzeitig die systolischen und diastolischen Zeitintervalle, wie z.B. PEP, LVET als Maß für die systolische Funktion, z.B. A2O als Maß für die diastolische Funktion, und dritter Herzton wie bei Herzinsuffizienz beobachtet, bzw. charakteristische Herzgeräusche, wie sie bei Vitien vorkommen, ermittelt werden.

Bei Verwendung eines Accelerometers -9b- kann die Öffnung -24- in der elektrisch leitenden Elektrode -23-wegfallen, weil die Beschleunigung auch durch die elektrisch leitende Elektrode -23- an den mechanischen Aufnehmer, z.B. Accelerometer -9b- weitergeleitet wird. Es ist daran gedacht, diesen Elektrodenkörper -11- entweder mittels einer klebenden Folie (nicht gezeigt, da Standard) oder eventuell in bekannter Weise mittels Saugelektrode am Körper anzubringen, wobei mit -15- eine elastische Saugglocke -15- gezeigt ist, die rund um den Elektrodenkörper -11- am untersuchten Körper eine dichte Lippe -16- bildet, sodass innerhalb der Saugelektrode ein Vakuum erzeugt werden kann.

Um möglichst optimale Signale zu erhalten, ist ein völlig vibrationsfreies Vakuum sehr von Vorteil. Die elastischen Saugglocken -15- sind über Saugleitungen -17- mit der Unterdruckquelle -18-, z.B. einer Saugpumpe verbunden wobei die Unterdruckquelle -18-entweder von vornherein vibrationsfrei sein könnte, wie dies z.B. bei einem Unterdruckbehälter mit großer Kapazität der Fall sein könnte, oder es ist zumindest ein entsprechend großes, stark dämpfendes Ausgleichsgefäß -19- oder mehrere Ausgleichsgefäße -19-, eventuell in Serie geschaltet, vorhanden. Eventuell finden sich zusätzlich Ventile -20-zwischen den elastischen Saugglocken -15- und dem/den Ausgleichsgefäß/en -19-, die immer nur dann öffnen, wenn die Unterdruckpumpe nicht eingeschaltet ist. So kann immer ein absolut vibrationsfreier Unterduck hergestellt werden, wobei z.B. auch die Signalgüte von elektrischen und mechanischen und optischen Abnehmern den optimalen Unterdruck rückgekoppelt adaptieren lassen könnte. Es kann dabei der Unterdruck so eingestellt werden, dass bei diesem die Signalgüte und das Signal-Rauschverhältnis von elektrischen, optischen, mechanischen und Temperatursensoren -35- optimal ist. Ein Ablassventil -21- für die rasche Beseitigung des Vakuums kann vorhanden sein, wobei dieses natürlich mit dem Ventil -20-kombiniert sein kann.

Um zu gewährleisten, dass der Unterdruck in den nicht an den Körper angelegten Elektroden nicht verloren geht, sind die Saugelektroden so ausgeführt, dass nur im angelegten Zustand an den Körper in der Saugkammer -15a-, die zwischen Elektrodenkörper -11- und elastischer Saugglocke -15- entsteht, wenn der Elektrodenkörper -11- die menschliche oder tierische Haut angelegt wird, ein Unterdruck entstehen kann. Dazu ist es notwendig, die Elektrode an den Körper zu pressen, wodurch sich eine Dichtlippe -22- zwischen dem Elektrodenkörper - 11- und der elastischen Saugglocke -15- öffnet, wie das schon 1984 beschrieben wurde (Lundbaeck, US4646747, 1984) . Sobald die Elektrode vom Körper entfernt wird, schließt sich durch die Eigenelastizität die Dichtlippe -22- um den Elektrodenkörper -11- und in der/den Saugleitungen -17- der angelegten und nicht angelegten Elektrode/n bleibt der Unterdruck aufrecht.

Wenn die elektrisch leitende Elektrode -23- z.B. Silberchloridelektrode eine z.B. asymmetrische Öffnung -24- für den mechanischen Aufnehmer -9-, z.B. ein Mikrofon -9a- oder ein Accelerometer -9b-, aufweist, hat dies den Vorteil, dass eine größere, geschlossene Elektrodenfläche für den elektrischen Kontakt mit der Haut zur Verfügung steht. Andererseits kann der mechanische Aufnehmer -9- ohne Veränderung der Position der Elektrode am Körper nur durch Drehung so platziert werden, dass der mechanische Aufnehmer -9-zwischen die Rippen des menschlichen Körpers zu liegen kommt, auch wenn der Hauptteil der elastischen Saugglocke -15- über den Rippen des menschlichen Körpers zu liegen kommen sollte. Durch Platzierung eines weiteren mechanischen Aufnehmers -9- in einer weiteren Elektrode am Thorax an einem Ort wo kein Herzschall oder Herzbewegung entsteht, z.B. V4r bis V6r, könnte auch der Hintergrundslärm oder die Hintergrundsbewegung am Thorax registriert und das Nutzsignal für diese Störung korrigiert werden.

Die Elektrodenkörperleitungen -29- von der elektrisch leitenden Elektrode -23- bzw. den mechanischen Aufnehmern -9- und/oder den Temperatursensoren -35- führen entweder z.B. zu einem Steckerweibchen -26- z.B. zu einem Multipolsteckerweibchen -26- und über die entsprechenden Kontakte -30- zu einem Stecker -25-, z.B. Multipolstecker -25-, oder auch direkt zum kombinierten EKG- Licht-, Mechano-. und Impedanz- und Temperatur-Analysator, abgekürzt ELMIT -27- bzw. zur CPU -27-. Die elektrischen Leitungen -31- sind dabei der Übersicht halber vielfach nur als einfache Leitungen gekennzeichnet, auch wenn mehr als eine elektrische Leitung -31- für die Funktion notwendig ist, auch sind nicht alle Leitungen mit der -31- gekennzeichnet, da die elektrische Verbindung zwischen den einzelnen Elementen in der Abbildung offensichtlich ist. Um nicht Rückstände von der Haut wie Schweiß, Haare oder Hautschuppen in die Saugleitungen -17- gelangen zu lassen, ist ein luftdurchlässiges Schwämmchen -28- vorgesehen, welches biologische Rückstände und Feuchtigkeit nicht durchläßt, und welches nach Wegstülpen der elastischen Saugglocke -15-ausgetauscht werden kann. Die Kontaktierung des z.B. Multipolsteckers -25- über das Multipolsteckerweibchens -26- zu den Elektrodenkörperleitungen -29- innerhalb des Elektrodenkörpers -11- stellt insofern eine Herausforderung dar, da die elastische Saugglocke -15- und damit auch der Elektrodenkörper -11- und damit auch der Multipolstecker -25-relativ klein sein müssen, damit ein guter Unterdruck aufgebaut werden kann. Trotzdem müssen die gesetzlich vorgeschriebenen elektrischen Sicherheitsabstände zwischen den Kontakten -30- eingehalten werden. Es wird daher vorgeschlagen, um den Multipolstecker - 25- ein elektrisch leitendes Rohr -32- anzubringen, das als zusätzlicher elektrischer Kontakt dient und welches gleichzeitig auch als Transportmedium für den Unterdruck dient und welches die Saugleitungen -17- gegenüber der elastischen Saugglocke -15- abdichtet und dabei ein leichtes Auswechseln der elastischen Saugglocke -15- und/oder des Elektrodenkörpers -11- ermöglicht. Das elektrisch leitende Rohr -32- ist dabei vom Multipolstecker -25- z.B. durch einen Isolierkörper -33- getrennt. Es muß natürlich sicher gestellt werden, daß das Vakuum innerhalb des Rohres mit der Saugleitung -17- innerhalb der elastischen Saugglocke in Verbindung steht, was z.B. durch Bohrung - 34- im Isolierkörper - 33- möglich wird. Im unteren Teil der Abbildung 3 ist der Elektrodenkörper -11- um 90 Grad gedreht gezeichnet, so dass die Ausformung der Saugleitung -17-, der Multipolstecker -25- und das Multipolsteckerweibchen -26- mit den Kontakten -30- hier besser sichtbar werden. Das elektrisch leitende Rohr -32- ist dabei strichliert gezeichnet, da es nicht in der Schnittebene der Abbildung liegt. Bei Ausprägung der Elektrode nur als elektrisch leitende Elektrode -23- ohne zusätzliche Aufnehmer am Elektrodenkörper -11- benötigt es natürlich keinen Multipolstecker -25- sondern nur einen einfach kontaktierten Stecker -25-. Der Stecker -25- bzw. das Steckerweibchen -26- dienen dazu, dass die elastische Saugglocke -15-ebenfalls leicht ausgetauscht werden kann. Der Stecker -25- und das Steckerweibchen -26-könnten irgendwo auch in der elektrischen Leitung -31- zum elektrischen oder elektronischen Schalter, z.B. Multiplexer -2- liegen. Die Stecker -25- müssen jedoch auch dann so schlank ausgeführt sein, dass sich die elektrischen Leitungen -31- mit dem Stecker -25- oder dem Steckerweibchen -26- gemeinsam mit dem in diesem Fall fix verbundenen Elektrodenkörper - 11- von der elastischen Saugglocke fädeln lassen, das heißt, dass die Saugglocke dann allein bzw. der Elektrodenkörper -11- allein mit dem daranhängenden Kabel leicht ausgetauscht werden können. Das heißt, dass der äußere Durchmesser des Steckers -25- auch dann nicht wesentlich größer sein darf, als die Ausnehmung -15b- in der elastischen Saugglocke -15-, in die in der Abbildung 3 das elektrisch leitende Rohr -32- eingeführt ist.

Es ist daran gedacht, während Phasen unveränderter Herztätigkeit Templates (Vorlagen) von allen gemessenen und errechneten Parametern zu generieren und zu speichern. Wenn dann die Untersuchung zu einem späteren Zeitpunkt wiederholt wird, können die Veränderungen der Parameter numerisch und graphisch als Trend aufgezeichnet werden. Dazu ist auch an die Verwendung von Normfeldern für die einzelnen Parameter gedacht. Wenn bei einer einzelnen Untersuchung mehrere Untersuchungsperioden z.B. nach physiologischen oder pharmakologischen Interventionen aneinander gereiht werden, und z.B. mehrere Templates derselben Parameter generiert werden, kann der Zeitgang der Änderung der Herz-Kreislauftätigkeit analysiert und der Trend aufgezeichnet werden. Wenn dann die Untersuchung nach einiger Zeit wiederholt wird, könnten die Trends der einzelnen Untersuchungen, die zu einem verschiedenen Zeitpunkt stattgefunden haben, dann überlagert, grafisch mit Datumsangabe dargestellt werden, umso leicht Veränderungen der Herz- und Kreislauftätigkeit zwischen den einzelnen Zeitpunkten der Untersuchungen zu erkennen. Auch hier ist an die Verwendung von Normfeldern für die einzelnen Parameter gedacht. Als Beispiele seien Veränderungen der ST Strecke oder des dz/dt während einer Belastungsuntersuchung angeführt. Die Signale für Anfang und Ende der Perioden können entweder händisch oder auch von einem anderen Gerät z.B. von einem Ergometer eingegeben werden. Speziell ist auch daran gedacht, alternative Herzschläge in separaten Templates zu analysieren, um so nicht nur den "electrical Alternans" sondern auch einen "mechanischen Alternans" zu generieren und zu analysieren, um so eine Fehlfunktion des Herzmuskels noch besser erkennen zu können. Die Messung der Änderung des Volumens nicht nur im Thorax sondern auch an zumindest einer Extremität (dZ oder dZ/dt) ermöglicht auch die Bestimmung eines concordanten oder discordanten Alternans des rechten und linken Ventrikels, da die Änderung des Volumens im Thorax mit dem Herzschlag vorwiegend durch die rechte Herzkammer, in der Extremität jedoch nur durch die linke Herzkammer bewirkt wird. Speziell wird man auch detektierte Extrasystolen dazu benützen, einen dadurch im Anschluß ausgelösten Alternans zu entdecken. Für die Auslösung von Alternans und auch für die generelle Analyse aller Signale, wie z.B. Herzzeitintervallen, Pulswellenlaufzeit, Volumsbeschleunigung durch dz/dt usw. ist auch an die Verwendung von Stimulationsmethoden des Kreislaufs, wie z.B. Erhöhung der Herzfrequenz durch Körperarbeit oder Pharmaka bzw. während der Erholungsphase von diesen Belastungen gedacht, weil dadurch eine noch bessere Beurteilung des Kreislaufs möglich ist.

Alle gewonnen Daten können in einem Speicher-40a-, dem Patienten zugeordnet, permanent gespeichert werden. So kann der Patient im Längsschnittuntersuchungen im Laufe der Zeit untersucht werden und Veränderungen gegenüber den Vorbefunden registriert und erkannt bzw. im Befund z.B. durch den Printer -40b- als Zahlen oder graphisch ausgegeben werden. Dies betrifft selbstverständlich nicht nur die elektrische Tätigkeit, wie Veränderungen des P-Wellen, R-Zacken- und T-Wellen- Vektors, Anomalien der p-Welle, des Kammerkomplexes und der Nachschwankung, der PQ-Zeit, der Dauer der Kammererregung, der Höhe der T-Welle, der QT-Dauer, QT-Dispersion und anderen Änderungen des EKGs, zusätzlich werden so auch Änderung der Impedanz mit dem Herzschlag und aller anderen oben aufgeführten Parameter wie z.B. Herzleistung, Herzinsuffizienzklasse (z.B. NYHA Klasse), geschätztes atriales natriuretisches Peptid, z.B. BNP, NT-proBNP, der lösliche ST2 receptor (sST2), Cardiotrophin, Adrenomedullin und/oder andere geschätzte biochemische Parameter für die Faserspannung des Herzmuskels bzw. für biomechanischen Stress, systolische Funktion, diastolische Funktion, Klappenfehler, Extrazellulärwasser, intrazelluläres Wasser, Gesamtkörperwasser, Muskelmasse, Fettmasse, Extrazellulär/Intrazellulärwasser-Verhältnis bzw. die Verteilung des Körperwassers und deren Teilkomponenten und deren Verhältnis zueinander in den einzelnen Körperteilen erkannt und als Zahlenwerte und/oder graphisch als Trend über die Zeit ausgegeben, sodass auf einen Blick eine Gefährdung des Patienten durch Änderung obiger Parameter erkannt werden kann. Die gewonnenen Rohdaten müssen dann zum Teil durch Kombination miteinander in komplexen Berechnungen, z.B. multiplen Regressionsgleichungen, verarbeitet werden, um die gewünschten Parameter zu errechnen. Als Beispiele seien nur die Kombination von Änderungen des dZ/dt max in verschiedenen Körpersegmenten mit einer Erkennung eines expandierten Extrazellulärwassers (ECW) z.B. eines erhöhten ECW in Relation zum intrazellulären Wasser (ICW) oder in Relation zum Total Body Water (TBW), also einer erhöhten ECW/ICW oder ECW/TBW Ratio genannt oder auch eine Änderung der Apexogrammkuve gemeinsam mit einer Änderung der systolischen und diastolischen Zeitintervalle, z.B. auch die rasche Relaxationszeit, nämlich die A2O-Zeit zur Erfassung einer gestörten diastolischen Funktion wie sie aus der Erkennung der Herztöne und des Apexocardiogramm ermittelt werden können. Auch die Veränderungen im Apexocardiogramm wie z.B. ein "mid systolic bulge" können zusätzlich zu den EKG Veränderungen zur Diagnose eines Herzinfarktes herangezogen werden. Auch Störungen des Percards, z.B. Pericarditis bzw. auch Klappenfehler z.B. Aortenstenose können auch über die Relation von a zu e-o-Welle im Apex Cardiogramm erkannt werden. Auch die erste Ableitung des Apexocardiogramm ermöglicht eine besonders genaue Beurteilung der Herzfunktion. Aber auch der Trainingszustand und eine Verbesserung der Herzkreislaufleistung läßt sich aus den angegebenen Parametern auch ohne Maximalbelastung über Regressionsgleichungen gut errechnen, so kann z.B. VO2max, die maximal zu leistende Wattanzahl und die körperliche Leistungsfähigkeit in Prozent der Norm z.B. mit Hilfe von multiplen Regressionsgleichungen bzw. auch neuronalen Netzen geschätzt werden, was bei der Trainingsberatung von Sportlern und Kranken hilft. Auch eine Veränderung der Muskelmasse in einzelnen Körpersegmenten und im Ganzkörper kann mit Hilfe der gewonnenen Daten erfasst werden.

Zur Erfassung von Über- oder Unterhydrierung bewährt sich besonders die Berechnung der Abweichung von TBW oder ECW oder ICW oder ECW/ICW Ratio von der zwischen der FM/kg Körpergewicht einerseits und TBW/kg Körpergewicht bzw. ECW/kg Körpergewicht bzw. ECW/ICW bei Gesunden ermittelten Regressionsgeraden. Um die Signalgüte aller Signale zu optimieren, ist auch daran gedacht, einerseits die Signale möglichst nahe an den Aufnehmern zu verstärken, z.B. in bekannter Weise mit Operationsverstärkern, die allfällige Einstreuungen bzw. Verfälschungen zu kompensieren, weiters sollten wo notwendig alle Leitungen mit aktiven Schirmen ausgestattet werden, außerdem ist, wo möglich, auch an eine frühe Digitalisierung der Signale nahe an den Aufnehmern gedacht, was etwaige Probleme bei der Signalgüte eliminieren helfen würde.

Fig. 4 zeigt eine andere Ausführung der Saugelektrode, bei der zwei verschiedene Stecker (= Steckverbindungen) -25-, -44- in dem von der elastischen Saugglocke -15- umgebenen Elektrodenkörper -11- den Kontakt mit den elektrisch leitenden Elektroden -23- aufnehmen. Aus Sicherheitsgründen muß nämlich zwischen den Elektrodenkörperleitungen -29-, die zur elektrisch leitenden Elektrode -23- und zum mechanischen Aufnehmer -9- führen, ein in den Normen festgelegter großer Sicherheitsabstand eingehalten werden, der sich anders kaum realisieren läßt. Wann immer von einem Stecker und einem Steckerweibchen in dieser Anmeldung die Rede ist, ist jedem Fachmann verständlich, dass die beiden Begriffe austauschbar gemeint sind, deswegen auch der Begriff Steckverbindung -25- eingeführt wird. Ein konischer Stecker -43- stellt beispielsweise den elektrischen Kontakt zum Steckerweibchen -26- her. Eine großflächige Ausführung des konischen Steckers -43-, z.B. mit vergoldeter Oberfläche gewährleistet auch einen guten elektrischen Kontakt im Falle der durch Feuchtigkeit ausgelösten Korrosion des konischen Steckers -43-. Eine Bohrung -34- im elektrisch leitenden Rohr -32- stellt dabei die Verbindung zur Saugkammer -15a- her. Ein zweiter Stecker -44-, z.B. ein Klinkenstecker, mit mehreren Kontakten -30- zum Steckerweibchen -26- ist z.B. im Elektrodenkörper - 11- befestigt und stellt die Verbindung zum mechanischen Aufnehmer -9- her. Zwei Ausnehmungen -15b - in der elastischen Saugglocke -15- dichten die Durchführung der zwei Stecker -25-, —44- gegenüber der Saugglocke -15-. Es ist jedem Fachmann verständlich, daß statt des zweiten Steckers --44-auch ein Steckerweibchen -26- im Elektrodenkörper -11- befestigt sein könnte und der zweite Stecker —44- durch die Ausnehmung -15b- in die Saugglocke -15- eingeführt werden könnte. Die tatsächlich gezeigte Ausführung hat aber den Vorteil, daß bei Austausch der Saugglocke - 15- der zweite Stecker —44- als beim Einführen des Elektrodenkörpers -11- in die elastische Saugglocke -15- der Führung durch die Ausnehmung -15b- dienen kann. Bei Vergleich der Abbildungen 2 und 4 zeigt sich schon, wieviel einfacher die Ausführung in Fig. 4 ist und um wieviel sicherer die gesetzlich notwendigen Sicherheitsabstände zwischen den elektrischen Leitungen eingehalten werden können. Dies speziell unter dem Umstand, daß es kaum verhindert werden kann, dass auch elektrisch leitende Flüssigkeit, die in bekannter Weise zur Verbesserung des elektrischen Kontaktes zwischen elektrisch leitender Elektrode -23- mit der Haut dient, in die Saugleitung -17- gelangt. Z.B. kann ein leicht austauschbares, luftdurchlässiges Schwämmchen -28-, ein Eindringen von Flüssigkeit bis zur Unterdruckquelle -18- verhindern. Die Steckverbinder -25-, 45- -26- können selbstverständlich auch durch Ausnehmungen -15b-, die an der dem untersuchten Körper visavis liegenden Fläche der Saugglocke -15- liegen, durchgeführt werden.

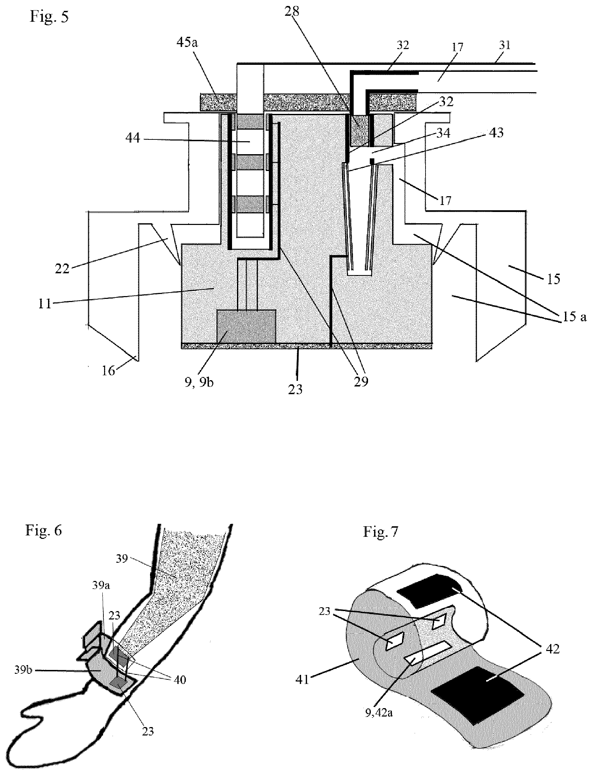

Eine weitere Ausführung, in Fig. 5 gezeigt, wäre, die Saugglocke -15- so zu gestalten, dass der Elektrodenkörper -11- an der dem untersuchten Körper abgewandten Seite frei aus der Saugglocke ragen kann, wobei das Elastomer den Elektrodenkörper -11- an der Zirkumferenz des Elektrodenkörpers -11- rundum dicht umgibt und luftdicht abschließt, wobei dann die Steckverbinder -25-, —44- -26- an der dem untersuchten Körper abgewandten freiliegenden Seite des Elektrodenkörpers - 11- angebracht werden. Am besten sind Stecker -25- und zweiter Stecker -44- durch einen Abstandhalter -45a- gemeinsam fixiert und als Winkelstecker ausgeführt. Auch hier können dann die Sicherheitsabstände gut eingehalten werden. Eine alternative, in den Abbildungen nicht gezeigte Ausführung dieser Saugelektrode wäre, die Elektrodenkörperleitungen -29- direkt mit der elektrisch leitenden Elektrode -23- und dem mechanischen Aufnehmer -9- z.B. dem Accelerometer -9b- direkt elektrisch zu verbinden und die elektrischen Leitungen -31- ferne dem Elektrodenkörper -11- bzw. der elastischen Saugglocke -15- steckbar zu machen. Diese Lösung ist besonders günstig wenn die Saugglocke entsprechend Fig. 5 ausgeführt ist, weil dann im Elastomer keine Ausnehmungen -15b- der elastischen Saugglocke -15- vorhanden sein müssen.

Es wurden auch verschiedene andere Elektrodenkonfigurationen getestet, bei diesen Untersuchungen hat sich gezeigt, dass auch nicht auf einem gemeinsamen Träger liegende Elektroden, wie z.B. eine Einzelklemme die auf den gegenüberliegenden getrennten Branchen jeweils eine einzelne elektrisch leitende Elektrode -23- trägt, von denen eine als Einspeiselektrode, die andere als Messelektrode verwendet wird, zu vergleichbaren Ergebnissen führt (Fig. 6). Es wird sich in diesem Fall das elektrische Feld -39-, gepunktet gezeichnet, zwar nicht komplett bis zur Messelektrode ausbreiten, jedoch wird das Prinzip der Vierpunktimpedanzmessung gewahrt und die fehlende Distanz zur Messelektrode auch in diesem Fall, wie in Fig. 2, als Ionenleiter -40- verwendet, da die hohe Ionenkonzentration im Organismus einen exzellenten elektrischen Leiter darstellt.

Fig. 7 zeigt eine weitere Ausführungsform einer gemeinsamen Elektrode für die Aufzeichnung des EKG und für die Emission und Registrierung von zusätzlichen physikalischen Parametern (z.B. einer oder mehrere der Parameter Strom, Spannung, Druck, Schall, Licht, Temperatur, Position). Mit -41- ist dabei schematisch entsprechend dem Aufgabenbereich der Erfindung eine EKG- Elektrode gezeigt, die Druck emittiert, nämlich eine aufblasbare Handgelenksmanschette -41-, der Klettverschluss ist mit -42-gekennzeichnet. Mit -23- sind die elektrisch leitenden Elektroden gekennzeichnet, die für die EKG Aufzeichnung und für die Impedanzmessung verwendet werden und die, versorgt mit Elektrodengel, durch das Aufblasen der Manschette gut an das Handgelenk gepresst werden. Mit -9- ist eine Vorrichtung zur Pulswellenanalyse gezeigt. Dabei könnte es sich z.B. um einen mechanischen Aufnehmer -9-, z.B. einen Drucksensor, z.B. Accelerometer -9b-handeln, das z.B. auch an der Wand einer flüssigkeitsgefüllten Blase -42a- angebracht ist. Diese Blase vergrössert die Fläche, an der der mechanische Aufnehmer -9-, oder ein anderer Drucksensor die Pulswelle registrieren kann, sodass eine genaue Platzierung der aufblasbaren Handgelenksmanschette -41- nicht notwendig ist und trotzdem die Pulswelle registiert werden kann. Gemeinsam mit den anderen Signalen kann dann die Pulswellenlaufzeit, der Augmentationsindex oder proximale und distale Compliance nach Watt und Burrus ( J Appl Physiol 40: 171- 176, 1976) und anderen Methoden registriert und analysiert werden und auch der arterielle Blutdruck z.B. nach der bekannten oszillometrischen Methode in kurzen Intervallen oder auch quasi-kontinuierlich bestimmt werden. Auch eine nicht aufblasbare Manschette könnte durch den Klettverschluß -42- oder anderen Verschluß genügend Auflagedruck der Sensoren bewirken, nur fällt dann die oszillometrische Blutdruckmessung fort.

In Ländern, in denen Saugelektroden aus hygienischen Bedenken nicht verwendet werden und in denen Einmalelektroden erwünscht sind, kann es sich bewähren, statt der Saugelektroden Klebelektroden zu verwenden. Bei den Extremitätenelektroden können dafür entweder Doppelektroden auf einer gemeinsamen Klebefolie -37a-, wie in Fig. 1 am Hals verwendet oder auch ein oder zwei einzelne Klebeelektroden -46- verwendet werden.

Eine weitere Version der Elektrodenkonfigurion ist in Fig. 8 gezeigt. Es handelt sich dabei um eine Universalelektrode, die sowohl als Saugelektrode oder auch als Klebelelektrode oder auch als Klemmelektrode ausgeführt werden kann. Mit -11- ist der Elektrodenkörper gezeigt der ein Accelerometer -9b- und einen Defibrillationsschutz -50- enthält (schematisch gezeichnet, der alle notwendigen Bauteile wie Widerstände und Kondensatoren enthält) wobei die Elektrodenkörperleitungen -29- mittels einer Druckknopfverbindung -45- an der elektrisch leitende Elektrode - 23 - angeklippt werden kann. Jede andere Art der leicht herstellbaren und trennbaren Verbindung (z.B. Steck- Klemm- , Schieberverbindung, federnde Klammer usw) statt der Druckknopfverbindung -45- könnte ebenfalls verwendet werden. Bei der elektrisch leitenden Elektrode -23- handelt es sich z.B. um eine Klebeelektrode -46- mit einem Elektrodengel getränktem Schwämmchen -47- mit außen liegender Klebefläche -48- oder um eine Saugelektrode mit umliegenden elastischen Saugglocke -15- wie in Fig. 8 gezeigt, Der Elektrodenkörper kann entweder in eine Saugelektrode eingebracht werden oder auch an konventionelle Klebeelektroden -46- oder auch Klemmelektroden -4-,-5-,-6- , wie für die Extremitätenelektroden vorgesehen, angeklippt werden, wobei die elastische Saugglocke - 15- natürlich wegfällt. Der Vorteil ist, dass mit nur einem Elektrodenkörper -11-, der Defibrillationsschutzschutz -50- und den mechanischen Aufnehmer -99a- z.B. das Accelerometer -9b- enthält, sowohl Halselektroden, Brustwandelektroden und Klemmelektroden bedient werden können, andererseits kann der Elektrodenkörper -11- bei der Kabelfertigung gleich in einem mit der Platine mitgefertigt, z.B. gegossen werden. Weiters ist die mit dem Gebrauch alternde elektrisch leitende Elektrode -23- auch bei Saug-Ausführung genauso leicht austauschbar wie bei den wegwerfbaren Klebeelektroden -46-. Zur Verstärkung der elektrisch leitenden Elektrode kann eine Grundplatte -51- vorgesehen sein. Zur Abdichtung der Druckknopfverbindung -45- von der umgebenden Feuchtigkeit kann eine Abdichtung, z.B. ein O-Ring -52- vorgesehen sein. Alle notwendigen Teile, wie Defibrillationsschutz -50- , Accelerometer -9b- und Druckknopfverbindung -45- können leicht auf einer gemeinsamen kleinen Platine -53- , die Platinenumrandung ist strichliert gezeichnet, untergebracht werden. Damit die Saugleitung -17- automatisch Anschluß an die Saugkammer -15a- findet, kann eine Nut des Elastomers --54- für die Aufnahme der elektrischen Leitungen -31- strichliert gezeichnet, in der elastischen Saugglocke -15-vorhanden sein.

Es ist eine möglichst trägheitsfreie Übertragung der mechanischen Schwingungen der Thoraxwand hervorgerufen durch 1., 2., oder auch 3. Herzton oder auch über interessierende Herzgeräusche, wie sie bei Vitien beobachtet werden, auf die Elektrode gewährleistet und trotzdem kann der mechanische Aufnehmer -9- immer wieder verwendet werden. Die elektrischen Leitungen -31- übertragen sowohl die elektrischen Signale als auch alle anderen physikalischen Signale an das EKG-Mechano-Impedanzgerät -27-. Wenn z.B. nur zwei der 10 Elektroden des 12 Kanal EKG ein Accelerometer -9b-, nämlich z.B. eine der Brustwandelektroden z.B. V1, V2, V4 einerseits und eine Extremitätenelektrode andererseits tragen, können die Herztöne und die Pulswelle aufgezeichnet werden, woraus sich eine exzellente Analyse der Kreislaufzeiten und anderer wichtiger Kreislaufparameter wie z.B. auch Pulswellenlaufzeit ergibt.

Eine solche Elektrode, genau angebracht z.B. über einer an der Oberfläche des Körpers liegenden Arterie, z.B. der Arteria radialis oder der Arteria temporalis oder der Arteria femoralis, Arteria tibialis posterior oder dorsalis pedis ist geeignet, die Pulswelle aufzuzeichnen und damit eine Analyse derselben z.B. mit Hilfe eines mathematsichen Modelles, z.B. des Windkesselmodells nach Watt und Burrus ( J Appl Physiol 40: 171- 176, 1976) oder mit Hilfe des Augmentationsindex ( J Appl Physiol 40: 171- 176, 1976) durchzuführen bzw. um die Pulswellenlaufzeit zu berechnen. Die gleichzeitige Aufzeichnung der Volumswelle mittels Impedanz und der Druckwelle mittels Accelerometer ermöglicht zusätzliche Einblicke in die Gefäßeigenschaften, wie z.B. der Compliance der oder des Füllungszustandes der Gefäße

Um bei Verwendung der rechten Beinelektrode für Impedanzmessungen durch die Verwendung des "right leg drive" keine Verfälschungen zu generieren, müssen entsprechende Maßnahmen bei der Gestaltung der Stromquelle eingehalten werden, die jedem Elektroniker gut vertraut sind.

Um die Reproduzierbarkeit der Thoraximpedanzmessungen zu verbessern. hat es sich auch bewährt aus den einzelnen Templates, gewonnen jeweils nur mittels Ableitung zwischen oberer Thoraxapertur und einer einzelnen Brustwandelektrode durch Mittelung, bevorzugt mit gleicher Wichtung der einzelnen Templates, ein gemeinsames Template zu ermitteln, weil damit besonders auch Variationen der Position der einzelnen Brustwandelektrode bei Untersuchungen zu unterschiedlichen Untersuchungszeitpunkten bei langzeitbetreuten Patienten, die nur schwer verhindert werden können, ausgeglichen werden können. Deswegen wird auch geraten, gut identifizierbare Positionen für die peripheren Elektroden festzulegen, z.B. sind dies proximal von Handgelenk, Knöchel sowie supraclaviculär.

Wie erwähnt, können zur Beseitigung von "Noise" bei der Registrierung von Herztönen und Herzgeräuschen durch die Elektroden V1 bis V6 z.B. die herzfernen Elektrodenpositionen V4r bis V6r. Benutzt werden. Dann könnte nämlich vom Signal der Herzaktionen das Signal des herzfernen Aufnehmers subtrahiert werden. Sollte es eine zeitliche Verschiebung zwischen den Signalen geben, könnte man zuerst auch noch eine Kreuzkorrelation der beiden Signale, bevorzugt unter Weglassen des Segments, das die Herzsignale trägt, durchführen und die beiden Signale so lange gegeneinander verschieben, bis die Übereinstimmung am besten und der Korrelationskoeffizient am größten ist. Erst danach sollte dann die Subtraktion der beiden wiederum kompletten Zeitreihen durchgeführt werden, sodass das Rauschen des Herzsignals größtmöglich eliminiert und möglichst nur das Nutzsignal übrigbleibt. Die herzfernen Elektroden am Brustkorb, speziell V5r und die Halselektrode kann auch in bekannter Weise dazu benutzt werden, um bipolare Brustwandableitungen z.B. CM5, CM5-, CC5 abzuleiten, um die Sensitivität der Ergometrie zur Entdeckung einer koronaren Herzkrankheit zu verbessern (Chaitman et al Circulation. 1978;57:71-79).

In Fig. 9 bis 13 wird die Ergebnis von Messungen mit Hilfe der hier beschriebenen Erfindung bei ca 120 gesunden und kranken Menschen gezeigt, wobei randomisiert ein Teil der untersuchten Menschen als Eichkollektiv und ein Teil als Evaluationskollektiv verwendet wurde. Für die Erstellung der Regressionsgleichungen und für die Eichung der gegenwärtigen Methode wurden sogenannte "Gold Standard Methoden" verwendet, wie z.B. BNP Bestimmung, echocardiographische Parameter wie Fractional Shortening, EF, TAPSE, E/A und e', Deuteriumverdünnung, Natriumbromidverdünnung, Ganzkörper DXA usw. Bei den Patienten handelt es sich um Patienten mit Herzkreislauferkrankungen, speziell mit chronischer Herzschwäche sowie Patienten mit Störungen des Flüssigkeitshaushaltes, wie Ödemen, Thoraxergüssen, Ascites.

Fig. 9 zeigt beispielhaft die gewonnenen Templates am Thorax und Bein bei einem Gesunden und bei einem Patienten mit Herzinsuffizienz. In Schwarz ist dabei das EKG Template dargestellt in Dunkelgrau das Thoraxtemplate des dZ/dt und in hellgrau das Beintemplate des dZ/dt. Beachte auch die Gipfelzeit, die für das Bein wegen der Volumslaufzeit verspätet beobachtet wird.

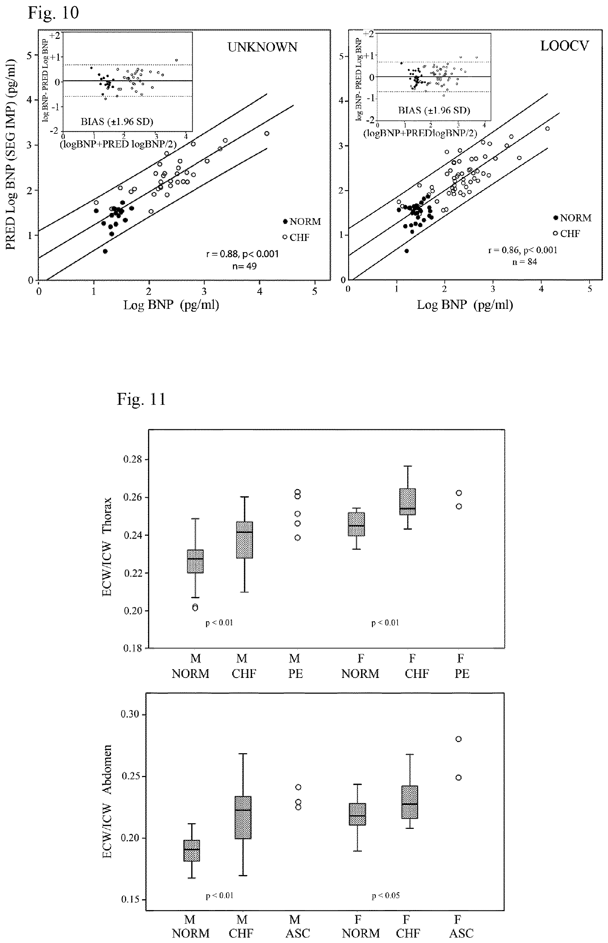

Fig. 10 zeigt die mittels multipler Regressionsgleichungen vorhergesagten BNP (brain natriuretic peptide) Werte. Die BNP Werte wurden logarithmiert, da die Verteilung des BNP einer logarithmischen Verteilung entspricht. Die Resultate einer multiplen Regressionsgleichung:

$$\text{Log BNP} = f(\text{dzt/dt Thorax}) + f(\text{dz/dt Beine oder Arme}) + f(\text{ECW/ICW Körpersegment bzw. Ganzkörper})$$

haben sich dabei z.B. besonders bewährt, wobei als Segmente herznahe und herzferne Segmente, nämlich das Thoraxsegment, Abdomensegment bzw. Arm- oder Beinsegmente evaluiert wurden.

Im linken Teil vorhergesagt an einem unbekannten Testkollektiv, welches nicht zur Berechnung herangezogen wurde (UNKNOWN), im rechten Teil der Abbildung wurde das LOOCV ("leave one out cross validation") Verfahren ebenfalls am unbekannten Kollektiv angewendet. Wie ersichtlich besteht eine sehr gute, klinisch brauchbare Vorhersage des BNP und der Herzinsuffizienz. Der Logarithmus des BNP kann dann wieder in die wahre Zahl umgerechnet werden. Das BNP ist deswegen besonders interessant, weil es sowohl bei Linksals auch bei Rechtsherzinsuffizienz und sowohl bei systolischer als auch diastolischer Herzinsuffizienz erhöht ist. Das Insert zeigt jeweils den bekannten Bland Altmann Plot. Mit NORM sind gesunde Kontrollen, Patienten sind mit PTS gekennzeichnet.

Tabelle 1 zeigt die Vorhersage einer Herzinsuffizienz mit Hilfe einer logistischen Regression mit den entsprechenden Sensitivitäten und Spezifitäten im Vergleich zu einem Patientenkollektiv ohne Herzinsuffizienz. Gezeigt ist die Originaltabelle aus dem SPSS Programm. Auch hier kann eine bemerkenswert gute Vorhersage einer Herzinsuffizienz erzielt werden.

Tabelle 1

| Klas sifikationstabelle | | | | | |
|---|---|---|---|---|---|
| Beobachtet | | | Voraussage | | |
| | | | Keine Herzschwäche | Herzschwäche | Prozentsatz korrekt |
| | | | 0,00 | 1,00 | |
| Schritt 1 | Kein Herzfehler | 0,00 | 39 | 3 | 92,9 |
| | Herzfehler | 1,00 | 4 | 28 | 87,5 |
| | Gesamtprozentsatz | | | | 90,5 |

Schnittwert ist 0,500
Schritt 1: dZ/dt Thorax, dZ/dt Beine

Goldstandardmethoden wie die Echocardiographie benötigt für diese Diagnosen einen ausgebildeten Kardiologen und eine Untersuchungsdauer von zumindest 20 bis 30 Minuten, wie das u.a. auch von der deutschen kardiologischen Gesellschaft gefordert wird.

Statt des BNP können selbstverständlich auch alle anderen Marker, die zur Abschätzung Herzleistung dienen, seien es Parameter für andere biochemische Marker ( z.B. NT-pro BNP, Adrenomedullin, Noradrenalin, Renin, Angiotensin, ADH, Aldosteron, Endothelin, usw, ob bereits verwendet oder noch nicht verwendet) Über die komplexe Kreislaufanalyse mit Beurteilung auch der peripheren Gefäße kann es möglich sein, auch Inflammationsmarker wie z.B. CRP. TNF-alpha, Interleukin usw vorherzusagen oder physikalische Marker, wie sie z.B. aus der Echocardiographie (z.B. EF, Fractional Shortenng, SV, E/A, E', PEP, LVET, Tei Index, TAPSE usw) oder dem Phonocardiogramm und/oder aus der Pulskurve (z.B. PEP, LVET) ermittelt werden. Auch andere aus dem ICG Signal extrahierte Parameter wie Steigungen oder Intervalle können zusätzlich in die Gleichungen eingehen. Auch die Kreislaufzeiten, z.B. ermittelt aus dem Phonocardiogramm, wie z.B. PEP und LVET können zusätzlich in die Gleichungen eingehen, wobei jeweils nur die hochsignifikanten Prediktoren (p< 0.01) in die Gleichung aufgenommen werden. In ähnlicher Weise kann z.B. die Maximalleistung des Patienten z.B. in Watt, VO2max, und die aerobe bzw. anaerobe Schwelle mit multiplen Regressionsgleichungen sehr genau geschätzt werden, wobei in diesem Fall auch die Muskelmasse der verschiedenen Körpersegmente, speziell der Beine, neben dem dZ oder dessen Ableitungen in die Regressionsgleichung eingehen kann.

Diese Regressionsgleichungen könnten z.B. lauten:

$$\text{VO2max (oder Maximalwatt, oder Lactat Turning Point I oder II)} =$$

f(dZ/dt in Ruhe) + f (dZ/dt Anstieg während submaximaler Belastung) + f(Muskelmasse Beine) + f(Gewicht) + f(Sex, numerisch) + f(Alter) usw, wobei auch hier nur die Parameter in die Regressionsgleichung eingehen, die signifikant zur Verbesserung der Vorhersage beitragen.

Zur Diagnose der peripheren Durchblutungsstörung kann sehr gut die Änderung der Impedanz der Extremitäten (Arme, Beine) mit dem Herzschlag verwendet werden. Bekanntlich ändert sich die Form des Rheogramms (=Änderung der Impedanz der Extremitäten) besonders der Beine bei peripherer arterieller Verschlußkrankheit (z.B. PAVK, Endangitis obliterans) insoferne, als die Gipfelhöhe sich ändern kann, der Gipfelzeitpunkt (gerechnet vom Beginn des Steilanstiegs) zu einem anderen Zeitpunkt kommt, der Gipfel runder wird und ein Verlust der dikroten Welle zu beobachten sein kann. Alle diese Änderungen können z.B. durch eine Fourieranalyse der Volumswelle berechnet und erkannt werden (siehe z.B. IEEE Trans Biomed Eng 30: 387-91. 1983, Eur J Appl Physiol 89: 384-86, 2003)

Es ist auch zu beachten, dass natürlich eine reduzierte Herzleistung und steife Gefäße, die einer Volumszunahme der Gefäße der Extremität.entgegenwirken, die Volumswelle ebenfalls verändern. Hier kann eine mathematische Korrektur der Volumswelle für eine reduzierte Herzleistung bzw. für Atherosklerose helfen, die PAVK noch besser zu erkennen. So könnte z.B. auch eine raschere Volumswellenlaufzeit, wie sie sich aus der zeitlichen Differenz des Beginns der anakroten Phase der Impedanzkurve am Herzen und an der Extremität ergibt, herangezogen werden, um eine Atherosklerose zu erkennen. Um die Pulswellenlaufzeit in Metern/Sekunde oder einer anderen Einheit ausgeben zu können, kann es sich bewähren, die Distanz zwischen Brustwandelektroden und Symphyse bzw. Ansatz des Beines zu messen, (z.B. auch durch die Laufzeitmessung zwischen den Elektroden) um dann die Zeitverzögerung zwischen Beginn der anakroten Welle des dZ am Thorax und am Bein auf die zurückgelegte Distanz zu normieren. Wenn eine Atherosklerose und eine Versteifung des Gefäßes vorliegt, wird naturgemäß auch die Gipfelhöhe des Volumssignals in der Extremität in der Höhe abgeschwächt. Durch Aufbringung von zusätzlichen Elektroden, z.B. unterhalb des Kniegelenkes (z.B. einer Klebe oder Klemmelektrode) kann auch differenziert werden, ob der Unterschenkel oder der Oberschenkel von der Verschlußkrankheit betroffen ist,

Eine weitere Möglichkeit der Analyse der Volumswelle ist ein mathematisches Modell, z.B. ein Windkesselmodell, wie es z.B. von Watt und Burrus für die periphere Pulswellenanalyse beschrieben wurde.

Das Verhältnis ECW zu TBW wird aus dem Verhältnis der Basisimpedanz (Grundimpedanz) bei einer niedrigen Frequenz zwischen theoretisch 0 KHz (ermittelt aus dem Cole-Cole Plot) und z B 10 kHz, z.B. 5 kHz, und einer höheren Frequenz (z.B. grösser als 100 kHz bis theoretisch ∞ kHz, ebenfalls ermittelt über den Cole Cole Plot, z.B. 400 kHz) bestimmt. Diskrete Frequenzen z.B. in der Gegend von 5 KHz und ca. 400 kHz genügen ebenfalls sehr gut um das Verhältnis ECW/TBW oder ECW/ICW zu errechnen. Bekannterweise wird das intrazelluläre Wasser (ICW) aus der Differenz Ganzkörperwasser (TBW) und Extrazelluläres Wasser (ECW) ermittelt. Um dem wahren Werten der in vivo Verhältnisse nahe zu kommen, können zusätzlich die spezifischen Widerstände, die Resistivitäten, von ECW, ICW bzw. TBW, (Zhu F et al. J Appl Physiol. 2006;100:717-24) in die Gleichungen eingebracht werden.

Fig. 11 zeigt auch, daß dieser Quotient sehr gut geeignet ist, Herzinsuffizienz (CHF), Lungenwasser, Pleuraergüsse (PE) und auch Ascites (ASC) bei Männern (M) und Frauen (F) vorherzusagen, was den diagnostischen Wert der vorgestellten Anmeldung weiter verbessert. Auch das schädliche viszerale Fett im Abdomen und ein- oder beidseitige Flüssigkeitsansammlungen in den Beinen, sei es Lymphödem, Beinvenenthrombosen oder beidseitige Beinödeme bei Herzinsuffizienz oder bei nephrotischem Syndrom können gut erkannt werden. Dazu können ebenfalls empirisch ermittelte Regressionsgleichungen oder auch durch mathematische Modelle ermittelte Regressionsgleichungen unter Einbeziehung von anatomischen Maßen, Längs- und Querdurchmesser bzw. Umfängen bzw. Fläche, bzw. Grösse, Gewicht und Sex (z.B. M=1, F=2) verwendet werden So kann auch sehr gut die Sarkopenie im Gesamtkörper oder in einem Körpersegment gut abgeschätzt werden. Auch hier werden jeweils nur die hochsignifikanten Prediktoren (p< 0.01) in die Gleichung aufgenommen. Bei beiden Rechenbeispielen können natürlich auch andere nicht lineare Verfahren, wie z.B. neuronale Netze für die Vorhersage verwendet werden.

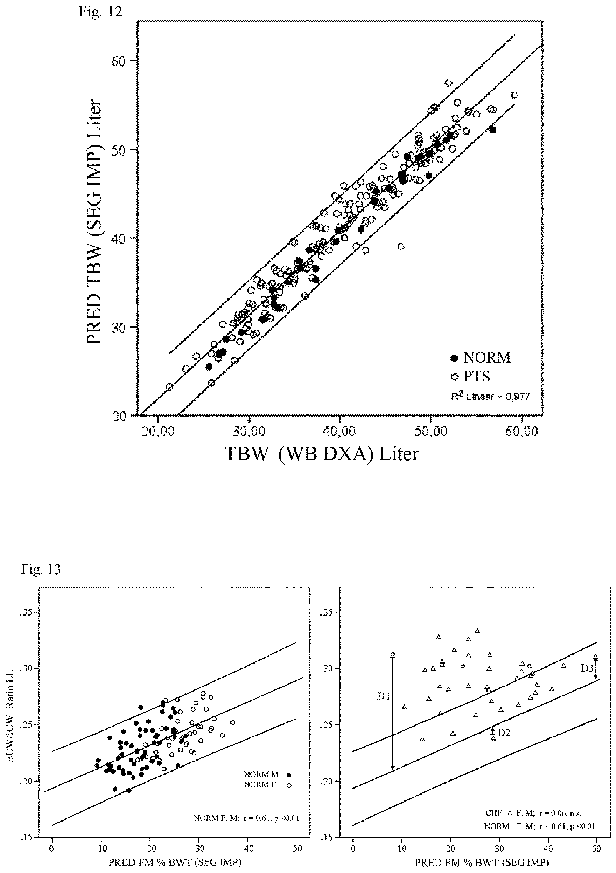

Neben der Berechnung des ECW/TBW oder ECW/ICW Verhältnisses gelingt mit der segmentalen Multfrequenzanalyse auch eine bemerkenswerte Vorhersage der Körperkompartimente, wie z.B. des Gesamtkörperwassers (TBW), des ECW und des ICW der "Lean Body Mass" (LBM) oder der "Fat Mass" (FM bzw. deren Abweichungen von der Norm wie beispielshaft in Fig. 12 gezeigt. Für diese Untersuchungen wurden insgesamt mehr als 120 Gesunde (NORM) und kranke Patienten (PTS) mit schweren Veränderungen des Salz-Wasserhaushaltes wie z.B. bei Herzinsuffizienz, Leber- und Nierenerkrankungen herangezogen. Die Regressionsgleichungen lauteten dabei z.B.:

TBW (oder TBW in % des Körpergewichts) oder ECW (oder ECW in % des Körpergewichts) oder TBW-Abweichung vom Sollwert oder ECW-Abweichung vom Sollwert oder Muskelmasse (oder Muskelmasse in % des Körpergewichts) oder Fettmasse (oder Fettmasse in % des Körpergewichts) = f(Z0 bei niedriger Frequenz, z.B. 0 oder 5 kHz und/oder bei hoher Frequenz, z.B. z 400 kHz oder dem theoretisch aus dem Cole-Cole Plot ermittelten ∞ kHz) Thorax + f(Z0 bei 5 und/oder 400 kHz)Abdomen + f(Z0 bei 5 und/oder 400 kHz oder ∞ kHz ) Arm + f(Z0 bei f(Z0 bei niedriger Frequenz, z.B. 0 oder5 kHz und/oder bei hoher Frequenz, z.B. z 400 kHz oder ∞ kHz)) Bein + f(Z0 bei niedriger Frequenz, z.B. 0 oder5 kHz und/oder bei hoher Frequenz, z.B. z 400 kHz oder ∞ kHz)) Ganzkörper und/oder Sex f(M/=1,F=2) und/oder f (Gewicht) und/oder f(Größe), wobei jeweils nur die hochsignifikanten Prediktoren (p< 0.01) in die Gleichung aufgenommen werden.

Gesunde Kontrollen sind in Fig. 12 als schwarze Kreise (NORM) , kranke Menschen "patients" (PTS) als weiße Kreise eingezeichnet. Die Kranken wiesen großteils schwere Störungen der Hydration mit Ödemen oder Exsiccose auf. Das Gewicht der untersuchten Personen betrug dabei zwischen 37 kg bei einer Anorexia nervosa und 155 kg bei schwerer morbider Adipositas. Trotz Einbeziehung dieser pathologischen Zustände gelingt die Vorhersage exzellent, wie wir mit Hilfe von Goldstandardmethoden wie der Deuteriumverdünnung und der Ganzkörper DXA (TBW-WB DXA) zur Bestimmung des TBW und der Natriumbromidverdünnung zur Bestimmung des ECW zeigen konnten. Auf der y-Achse findet sich das mittels segmentaler Impedanz vorhergesagte TBW (PRED TBW (SEG IMP)). Auch die Streuung für kranke Menschen ist nicht wesentlich grösser als für gesunde Kontrollen. Derartig gute Ergebnisse für kranke Menschen sind in der Literatur nicht bekannt, was die Bedeutung dieser Methode für den klinischen Alltag unterstreicht.

Fig. 13 zeigt auf der x-Achse die Beziehung zwischen aus der segmentalen Impedanz vorhergesagtem Körperfett = "fat mass", (FM), ausgedrückt als Prozentsatz des Körpergewichtes, wobei dieser Parameter bereits ausschließlich mit Hilfe der segmentalen Impedanzmessung, wie hier vorgestellt, errechnet wurde. Auf der y-Achse ist das Verhältnis von ECW/ICW in einem herzfernen Segment z.B. im linken Bein, "left leg" (LL) dargestellt. Links in der Fig. 13 sind gesunde Männer (NORM M) als schwarze Kreise, gesunde Frauen (NORM F) als weiße Kreise dargestellt. Wie ersichtlich haben bei dieser Darstellung gesunde Frauen eine grössere ECW/ICW Ratio, offensichtlich weil sie einen höheren Prozentsatz an Körperfett haben. In der Abbildung steht NORM für normal=gesund. Die signifikante Regressionslinie ist ebenfalls eingezeichnet. Im rechten Teil der Abbildung sind männliche und weibliche Patienten mit chronischer Herzschwäche, "chronic heart failure" (CHF) als weiße Dreiecke gekennzeichnet. Wie ersichtlich hat ein hoher Prozentsatz dieser Patienten wie erwartet einen beträchtlichen "Fluid Overload" im Extrazellulärraum, ein Teil jedoch hat das "Trockengewicht" im Rahmen der Behandlung erreicht. Die senkrechte Distanz D zur Regressionslinie zeigt den Grad der Über- oder Unterhydrierung. D1 zeigt einen Patienten mit sehr grosser Überhydrierung, D2 und D3 kennzeichnen 2 Patienten mit normalem "Trockengewicht". Auch eine Umrechnung von z.B. D1, D2, D3, in Liter Überschuss oder Fehlmenge ECW ist damit möglich.

So gelingt erstmals klar die Feststellung der Über- oder Unterhydrierung des Organismus, was vorher mittels Impedanzanalyse und auch mit anderen Methoden nicht möglich war. Dies ist z.B. auch von grosser Bedeutung für die Behandlung von Patienten mit z.B. CHF, Nierenkranken, chronischer Niereninsuffizienz, Dialysepatienten, Lebererkrankungen, Schwangerschaftsgestose oder auch Zustände von Exsiccose. Statt der Fettmasse kann in dieser Grafik auf der x-Achse natürlich das Gegenteil, nämlich ein Parameter für die "Non Fat Mass" wie der Prozentsatz des Körpergewichtes von Ganzkörperwasser, ( TBW) , Lean Body Mass (LBM) verwendet werden, wobei die Regression natürlich keine positive sondern eine negative Steigung aufweist.

Die Erfindung ist in den Ansprüchen definiert.

## Patentansprüche

1. EKG-Gerät (27) mit EKG-Ableitungs-Elektroden (V1-V6, V4r-V6r, 3, 5, 6), die zumindest Extremitätenelektroden (5, 6) umfassen, wobei zumindest eine der EKG-Ableitungs-Elektroden (3, 5, 6) zusätzlich zu ihrer EKG-Ableitungsfunktion zur Emission und/oder zum Empfang von zusätzlichen physikalischen Signalen, insbesondere Strom, Spannung, Druck, Schall, Licht, Temperatur, Position, ausgebildet ist, wobei die EKG-Ableitungs-Elektroden (V1-V6, V4r-V6r, 3, 5, 6) weiters ausgebildet sind, voneinander beabstandet am menschlichen Körper angeordnet zu werden und dabei zwischen einander ein Wechselstromfeld aufzubauen, wobei zusätzlich zu den EKG-Ableitungs-Elektroden zumindest eine an der oberen Thoraxapertur, insbesondere am Kopf, Hals, Nacken, an der Schulter oder den Armen anordenbare Elektrode (3, 6, 6a) vorgesehen ist, die zur Einspeisung und/oder Messung eines Wechselstroms ausgebildet ist und das EKG-Gerät (27) so ausgeprägt ist, dass es auch die Änderung der Impedanz in Abhängigkeit von dem Herzschlag in zumindest einem herznahen und zumindest einem herzfernen Segment registriert, wobei die Elektroden im am Körper angeordneten Zustand zur Induzierung einer Ionenleitung im Körper ausgebildet sind, wobei das EKG-Gerät (27) dazu konfiguriert ist, jene Teile des Körpers, die nicht von einem Wechselstromfeld durchströmt sind, als Ionenleiter zu verwenden, wobei das EKG-Gerät (27) dazu ausgeprägt ist, die Stromeinspeisung zwischen einer an einem Bein anordenbaren ersten distalen Beinelektrode (5) und einer am anderen Bein anordenbaren zweiten distalen Beinelektrode (5) zu schalten und die Impedanz und die Änderung der Impedanz in Abhängigkeit vom Herzschlag alternierend zwischen der an der oberen Körperapertur anordenbaren Elektrode (3, 6a) und der ersten distalen Beinelektrode (5) bzw. der an der oberen Körperapertur anordenbaren Elektrode (3, 6a) und der zweiten distalen Beinelektrode (5) zu messen.

2. EKG-Gerät nach Anspruch 1, **dadurch gekennzeichnet, dass** zumindest eine der EKG-Ableitungs-Elektroden einen Elektrodenkörper (11) aufweist, der einen als Accelerometer (9b) ausgebildeten Aufnehmer (9) für mechanische Schwingungen aufweist.

3. EKG-Gerät nach Anspruch 2, **dadurch gekennzeichnet, dass** innerhalb des Elektrodenkörpers (11) eine Aussparung vorgesehen ist, in der der mechanische Aufnehmer (9) asymmetrisch gegenüber einer elektrisch leitenden Elektrode (23) untergebracht ist.

4. EKG-Gerät nach Anspruch 2 oder 3, **dadurch gekennzeichnet, dass** es für die Auswertung der vom mechanischen Aufnehmer (9) gelieferten Ausgangssignale zumindest einen, vorteilhaft zumindest zwei Frequenzfilter aufweist, und dazu ausgebildet ist zumindest einen, vorteilhaft zumindest zwei, gefilterte Frequenzbereiche der Ausgangssignale separat zu analysieren, wobei die Filter bevorzugt als Bandpassfilter mit einem Frequenzband zwischen 50 und 1000 Hz und als Bandbassfilter mit einem Frequenzband zwischen 0,1 Hz und 70 Hz ausgebildet sind.

5. EKG-Gerät nach einem der Ansprüche 2 bis 4, **dadurch gekennzeichnet, dass** es dazu konfiguriert ist, aus einer mittels der Elektroden ermittelten Impedanzänderung nach der Zeit dz/dt am Thorax und/oder den Beinen und/oder von dem zumindest einen Aufnehmer (9) für mechanische Schwingungen gewonnenen physikalischen Messsignalen die systolische und/oder diastolische Funktion und/oder deren Störungen zu errechnen und anzuzeigen.

6. EKG-Gerät nach einem der vorhergehenden Ansprüche, **dadurch gekennzeichnet, dass** es dazu konfiguriert ist, die körperliche Leistungsfähigkeit, z.B. VO2max, maximale Wattanzahl, Leistungsfähigkeit in Prozent der Norm auch ohne Maximalbelastung aus den gemessenen Parametern, z.B. PEP, LVET, dZ/dtmax, oder geschätzten Parametern wie zB Muskelmasse, aus den Parametern des Accelerometers (9b), z.B. PEP, LVET, usw. mittels Regressionsgleichungen bzw. neuronalen Netzen rechnerisch zu schätzen.

7. EKG-Gerät nach einem der vorhergehenden Ansprüche, **dadurch gekennzeichnet, dass** es Brustwandelektroden nach Wilson aufweist.

8. EKG-Gerät nach einem der vorhergehenden Ansprüche, **dadurch gekennzeichnet, dass** das EKG-Gerät dazu ausgeprägt ist, die Stromeinspeisung zwischen der an der oberen Körperapertur anordenbaren Elektrode und der an einem Bein anordenbaren ersten distalen Beinelektrode zu schalten und die Impedanz und die Änderung der Impedanz in Abhängigkeit vom Herzschlag zwischen der an der oberen Körperapertur anordenbaren Elektrode und der am zweiten Bein anordenbaren zweiten distalen Beinelektrode oder zwischen der ersten und der zweiten distalen Beinelektroden zu messen.

9. EKG-Gerät nach einem der vorhergehenden Ansprüche, **dadurch gekennzeichnet, dass** die Elektrode an der Thoraxapertur als eine Doppelelektrode (1, 3) auf einem gemeinsamen Träger (37, 37a) ausgebildet ist.

10. EKG-Gerät nach einem der vorhergehenden Ansprüche, **dadurch gekennzeichnet, dass** ein Wechselstrom bei mehreren Frequenzen, bevorzugt bei zwischen 1 und 10 kHz, bei zwischen 30 und 200 kHz und bei zwischen 200 und 1000 kHz, in die Elektroden einspeisbar ist.

11. EKG-Gerät nach Anspruch 10, **dadurch gekennzeichnet, dass** die Schaltvorrichtung (2) dazu ausgebildet ist elektrischen Strom an einer an den unteren Extremitäten anordenbaren Elektrode und an der an der oberen Thoraxapertur anordenbaren Elektrode einzuspeisen und dass das EKG-Gerät dazu ausgeprägt ist die Impedanz zwischen der an der oberen Thoraxapertur anordenbaren Elektrode und einer der an der unteren Thoraxapertur anordenbaren Elektrode zu messen, wobei die an der unteren Thoraxapertur anordenbare Elektrode vorzugsweise als Brustwandelektrode (V3-V6, V3r-V6r) ausgebildet ist.

12. EKG-Gerät nach einem der vorhergehenden Ansprüche, **dadurch gekennzeichnet, dass** zumindest einige der Elektroden als Vakuumelektroden ausgebildet sind, wobei optional eine Vorrichtung vorgesehen ist, die die Höhe des Unterdrucks an den Saugelektroden in Hinblick auf ein ermitteltes physikalisches Signal, z.B. ein mechanisches, akustisches, elektrisches oder optisches Signal regelt und/oder optimiert.

13. EKG-Gerät nach einem der vorhergehenden Ansprüche, **dadurch gekennzeichnet, dass** mittels einer Recheneinheit mehrere von den Elektroden erfasste Messkurven zu einer Template-Messkurve übereinander gelegt werden.

14. EKG-Gerät nach einem der vorhergehenden Ansprüche, **dadurch gekennzeichnet, dass** das EKG-Gerät dazu ausgebildet ist, die Änderung der Impedanz in Abhängigkeit vom Herzschlag an zumindest zwei Körpersegmenten zu messen und daraus und optional zusätzlich aus einem Maß für das Extrazellulärvolumen, z.B. dem Verhältnis von Extrazellulärwasser zu Ganzkörperwasser oder dem Verhältnis Extrazellulärwasser zu Intrazellulärwasser, die Herzleistung und den Grad einer Herzschwäche, und/oder einen biochemischen Parameter für den biomechanischen Stress von Herzmuskelzellen und/oder eine Wasseransammlung, und/oder eines der atrialen natriuretischen Peptide, z.B. BNP, NT-proBNP oder Adrenomedullin, zu errechnen.

15. EKG-Gerät nach einem der vorhergehenden Ansprüche, **dadurch gekennzeichnet, dass** es eine Recheneinheit umfasst, die dazu ausgebildet ist, eine Relation zwischen einem Parameter für den Körperfettanteil oder den Gesamtkörperwasseranteil (TBW) oder die Lean Body Mass (LBM), jeweils bezogen auf das Gesamtkörpergewicht, und einem Parameter für das extrazelluläre Wasser (ECW), z.B. das Verhältnis ECW/TBW oder ECW/ICW aus den segmentalen Impedanzen bei niedrigen und hohen Frequenzen für herznahe und/oder herzferne Segmente bzw. für Ganzkörper zu berechnen und dieses Verhältnis in Relation zu den Normwerten und/oder einer Regressionslinie auszugeben.

16. EKG-Gerät nach einem der vorhergehenden Ansprüche, **dadurch gekennzeichnet, dass** es einen Speicher (40a) für die Speicherung der ermittelten und errechneten Daten umfasst, und dass es eine Schnittstelle zu einer Anzeige und/oder einem Drucker aufweist, um die aktuellen Werte und die Vorwerte der erhobenen elektrischen und physikalischen Parameter über die Zeit als Zahlen oder graphisch mit Normfeldern an einer Anzeige darzustellen und/oder an einem Drucker auszudrucken, wobei das EKG-Gerät vorzugsweise dazu ausgebildet ist, bei vorhandenen mehreren Messphasen während einer Einzeluntersuchung die Ergebnisse der Messphasen als Trend graphisch darzustellen und bei einer wiederholten Untersuchung zu einem späteren Zeitpunkt die Messphasen der verschiedenen Untersuchungen übereinander gelagert mit Zeitbezeichnung graphisch darzustellen.

17. EKG-Gerät nach einem der vorhergehenden Ansprüche, **dadurch gekennzeichnet, dass** ein Elektrodenkörper mit einer leicht lösbaren Verbindung, z.B. einer Druckknopfverbindung (45) ausgestattet ist, die zu einer Elektrode führt, die vorzugsweise als Klebeelektrode (46) oder Klemmelektrode (1) oder elastische Bandelektrode ausgebildet ist, wobei der Elektrodenkörper in eine Saugglocke (15) eingeführt werden kann.

18. EKG-Gerät nach einem der vorhergehenden Ansprüche, **dadurch gekennzeichnet, dass** zumindest eine der EKG-Ableitungs-Elektroden (23) mit einer aufblasbaren Manschette und mit dem mechanischen Aufnehmer (9) für mechanische Schwingungen in Form eines Accelerometers (9b) ausgestattet ist, wobei der mechanische Aufnehmer (9) für die Positionierung an einer darunter liegenden Arterie ausgestaltet ist, wobei der mechanische Aufnehmer (9) vorzugsweise in oder an einer flüssigkeitsgefüllten Blase (42a) ausgebildet ist.

19. EKG-Gerät nach einem der vorhergehenden Ansprüche, **dadurch gekennzeichnet, dass** elastisch verbundene Branchen einer Elektrode vorhanden sind, von denen eine Branche für Stromeinspeisung (39a) und eine Branche als Branche für EKG und Impedanzmesselektrode (39b) ausgeprägt ist, wobei die Distanz der beiden Branchen im an den Körper angelegten Zustand als Ionenleiter (40) ausgeprägt ist.

## Claims

1. An ECG device (27) with ECG lead electrodes (V1-V6, V4r-V6r, 3, 5, 6) comprising at least limb electrodes (5, 6), wherein at least one of the ECG lead electrodes (3, 5, 6) is designed, in addition to its ECG lead function, for the emission and/or for receiving additional physical signals, in particular current, voltage, pressure, sound, light, temperature, position, wherein the ECG lead electrodes (V1-V6, V4r-V6r, 3, 5, 6) are furthermore designed for being arranged at the human body spaced apart from each other, thereby building up an alternating current field between each other, wherein, in addition to the ECG lead electrodes, at least one electrode (3, 6, 6a) positionable at the upper thorax aperture, in particular at the head, neck, the back of the neck, at the shoulder or the arms, is provided, which is designed for the supply and/or measurement of an alternating current, and the ECG device (27) is developed such that it also records the change in impedance as a function of the heartbeat in at least one segment close to the heart and at least one segment remote from the heart, wherein the electrodes in the state of being arranged at the body are designed for inducing an ion conduction in the body, the ECG device (27) being configured for using those parts of the body as ion conductors which are not passed through by an alternating current field, the ECG device (27) being developed for switching the current supply between a first distal leg electrode (5) positionable at one leg and a second distal leg electrode (5) positionable at the other leg and for measuring the impedance and the change in impedance as a function of the heartbeat alternately between the electrode (3, 6a) positionable at the upper body aperture and the first distal leg electrode (5) or, respectively, the electrode (3, 6a) positionable at the upper body aperture and the second distal leg electrode (5).

2. An ECG device according to claim 1, **characterized in that** at least one of the ECG lead electrodes comprises an electrode body (11) which exhibits a sensor (9) designed as an accelerometer (9b) for mechanical vibrations.

3. An ECG device according to claim 2, **characterized in that** a recess is provided within the electrode body (11), in which recess the mechanical sensor (9) is accommodated asymmetrically opposite to an electrically conductive electrode (23).

4. An ECG device according to claim 2 or 3, **characterized in that**, for the evaluation of the output signals provided by the mechanical sensor (9), it comprises at least one, advantageously at least two, frequency filters and is designed for analyzing at least one, advantageously at least two, filtered frequency ranges of the output signals separately, wherein the filters are preferably designed as band-pass filters with a frequency band of between 50 and 1000 Hz and as band-pass filters with a frequency band of between 0.1 Hz and 70 Hz.

5. An ECG device according to any of claims 2 to 4, **characterized in that** it is configured for calculating and indicating the systolic and/or diastolic function and/or disorders thereof from a change in impedance after the time dz/dt at the thorax and/or the legs, as determined by means of the electrodes, and/or from physical measurement signals obtained by the at least one sensor (9) for mechanical vibrations.

6. An ECG device according to any of the preceding claims, **characterized in that** it is configured for arithmetically estimating the physical performance, e.g., V02max, the maximum wattage, the performance in percent of the standard, also without a maximum load, from the measured parameters, e.g., PEP, LVET, dZ/dtmax, or estimated parameters such as, e.g., muscle mass, from the parameters of the accelerometer (9b), e.g., PEP, LVET, etc., by means of regression equations and, respectively, neural networks.

7. An ECG device according to any of the preceding claims, **characterized in that** it comprises chest wall electrodes according to Wilson.

8. An ECG device according to any of the preceding claims, **characterized in that** the ECG device is developed for switching the current supply between the electrode positionable at the upper body aperture and the first distal leg electrode positionable at a leg and for measuring the impedance and the change in impedance as a function of the heartbeat between the electrode positionable at the upper body aperture and the second distal leg electrode positionable at the second leg or between the first and the second distal leg electrodes.

9. An ECG device according to any of the preceding claims, **characterized in that** the electrode at the thorax aperture is designed as a double electrode (1, 3) on a common carrier (37, 37a).

10. An ECG device according to any of the preceding claims, **characterized in that** an alternating current is feedable into the electrodes at several frequencies, preferably at between 1 and 10 kHz, at between 30 and 200 kHz and at between 200 and 1000 kHz.

11. An ECG device according to claim 10, **characterized in that** the switching apparatus (2) is designed for supplying electric current at an electrode positionable at the lower limbs and at the electrode positionable at the upper thorax aperture and that the ECG device is developed for measuring the impedance between the electrode positionable at the upper thorax aperture and an electrode positionable at the lower thorax aperture, wherein the electrode positionable at the lower thorax aperture is preferably designed as a chest wall electrode (V3-V6, V3r-V6r).

12. An ECG device according to any of the preceding claims, **characterized in that** at least several of the electrodes are designed as vacuum electrodes, wherein, optionally, a device is provided which controls and/or optimizes the level of the negative pressure at the suction electrodes with regard to a detected physical signal, for example, a mechanical, acoustic, electric or optical signal.

13. An ECG device according to any of the preceding claims, **characterized in that** several measuring curves detected by the electrodes are laid on top of each other by means of an arithmetic unit in order to form a template measuring curve.

14. An ECG device according to any of the preceding claims, **characterized in that** the ECG device is designed for measuring the change in impedance as a function of the heartbeat at at least two body segments and for calculating therefrom and optionally additionally from a measure for the extracellular volume, e.g., the ratio of extracellular water to total body water or the ratio of extracellular water to intracellular water, the cardiac output and the level of a cardiac insufficiency, and/or a biochemical parameter for the biomechanical stress of myocardium cells and/or a water accumulation and/or one of the atrial natriuretic peptides, e.g., BNP, NT-proBNP or adrenomedullin.

15. An ECG device according to any of the preceding claims, **characterized in that** it comprises an arithmetic unit designed for calculating a relation between a parameter for the body fat percentage or the total body water percentage (TBW) or the lean body mass (LBM), in each case based on the total body weight, and a parameter for the extracellular water (ECW), e.g., the ratio ECW/TBW or ECW/ICW, from the segmental impedances at low and high frequencies for segments close to the heart and/or remote from the heart or, respectively, for the total body and for displaying said ratio in relation to the standard values and/or a regression line.

16. An ECG device according to any of the preceding claims, **characterized in that** it comprises a memory (40a) for storing the determined and calculated data and that it has an interface to a display and/or a printer in order to illustrate the current values and the previous values of the electrical and physical parameters which have been gathered over time as numbers or graphically with standard panels at a display and/or to print them at a printer, wherein the ECG device is preferably designed, if several measurement phases are provided, for graphically depicting the results of the measurement phases as a trend during an individual examination and, in case of a repeated examination at a later point in time, for graphically depicting the measurement phases of the different examinations in an overlaid state with a time identification.

17. An ECG device according to any of the preceding claims, **characterized in that** an electrode body is provided with an easily detachable connection, for example, a push-button connection (45), which leads to an electrode which is preferably designed as an adhesive electrode (46) or a clamping electrode (1) or an elastic band electrode, wherein the electrode body can be inserted into a suction cup (15).

18. An ECG device according to any of the preceding claims, **characterized in that** at least one of the ECG lead electrodes (23) is equipped with an inflatable cuff and with the mechanical sensor (9) for mechanical vibrations in the form of an accelerometer (9b), wherein the mechanical sensor (9) is designed for being positioned at an underlying artery, the mechanical sensor (9) preferably being formed in or at a liquid-filled bubble (42a).

19. An ECG device according to any of the preceding claims, **characterized in that** elastically joined branches of an electrode are provided, one of the branches being developed for the current supply (39a) and one branch as a branch for ECG and the impedance measuring electrode (39b), wherein the distance of the two branches is developed as an ion conductor (40) in the state of being applied to the body.

**Revendications**

1. Appareil ECG (27) avec des électrodes de dérivation d'ECG (V1-V6, V4r-V6r, 3, 5, 6) qui comprennent au moins des électrodes d'extrémité (5, 6), dans lequel au moins une des électrodes de dérivation d'ECG (3, 5, 6) est conçue, en plus de sa fonction de dérivation d'ECG, pour émettre et/ou pour recevoir des signaux physiques supplémentaires, en particulier de courant, de tension, de pression, de son, de lumière, de température ou de position, dans lequel les électrodes de dérivation d'ECG (V1-V6, V4r-V6r, 3, 5, 6) sont en outre conçues pour être disposées à distance les unes des autres sur le corps humain et pour créer ainsi entre elles un champ de courant alternatif, dans lequel il est prévu, en plus des électrodes de dérivation d'ECG, au moins une électrode (3, 6, 6a) pouvant être placée au niveau de l'ouverture supérieure du thorax, en particulier de la tête, du cou, des épaules ou des bras, qui est conçue pour injecter et/ou mesurer un courant alternatif, cet appareil ECG (27) étant développé pour enregistrer également la variation d'impédance en fonction du battement cardiaque dans au moins un segment proche du coeur et au moins un segment éloigné du coeur, les électrodes étant configurées pour induire un courant ionique dans le corps lorsqu'elles sont disposées sur le corps, cet appareil ECG (27) étant configuré pour utiliser toutes les parties du corps qui ne sont pas traversées par un champ de courant alternatif comme des conducteurs ioniques et cet appareil ECG (27) étant développé pour enclencher l'alimentation électrique entre une première électrode de jambe distale (5) placée sur une jambe et une deuxième électrode de jambe distale (5) placée sur l'autre jambe et pour mesurer l'impédance et la variation de l'impédance en fonction du battement cardiaque entre l'électrode (3, 6a) placée au niveau de l'ouverture supérieure du corps et la première électrode de jambe distale (5) ou entre l'électrode (3, 6a) placée au niveau de l'ouverture supérieure du corps et la deuxième électrode de jambe distale (5).

2. Appareil ECG selon la revendication 1, **caractérisé en ce qu'**au moins une des électrodes de dérivation d'ECG comporte un corps d'électrode (11) qui présente un capteur (9) configuré comme un accéléromètre (9a) pour les vibrations mécaniques.

3. Appareil ECG selon la revendication 2, **caractérisé en ce qu'**à l'intérieur du corps d'électrode (11) est prévue une cavité dans laquelle le capteur mécanique (9) est logé de façon asymétrique par rapport à une électrode électro-conductrice (23).

4. Appareil ECG selon la revendication 2 ou 3, **caractérisé en ce qu'**il comporte pour l'évaluation des signaux de sortie fournis par le capteur mécanique (9) au moins un, de façon avantageuse au moins deux filtres de fréquence, et est configuré pour analyser séparément au moins un, de façon avantageuse au moins deux plages de fréquence filtrées des signaux de sortie, les filtres étant configurés de préférence comme des filtres passe-bande avec une plage de fréquence de 50 à 1000 Hz et des filtres passe-bas avec une plage de fréquence de 0,1 Hz à 70 Hz.

5. Appareil ECG selon une des revendications 2 à 4, **caractérisé en ce qu'**il est configuré pour calculer et pour indiquer, à partir d'une variation d'impédance après le délai dz/dt déterminée au moyen des électrodes sur le thorax et/ou les jambes et/ou des signaux de mesure physiques acquis par l'au moins un capteur (9) pour les vibrations mécaniques, les fonctions systolique et/ou diastolique et/ou leurs perturbations.

6. Appareil ECG selon une des revendications précédentes, **caractérisé en ce qu'**il est configuré pour estimer arithmétiquement la performance physique, p. ex. VO2max, le nombre de watts maximum, la performance en pourcentage de la norme même sans charge maximale, à partir des paramètres mesurés, p. ex. PEP, LVET, dZ/dtmax, ou de paramètres estimés comme p. ex. la masse musculaire, à partir des paramètres de l'accéléromètre (9b), p. ex. PEP, LVET, etc., au moyen d'équations de régression ou de réseaux neuronaux.

7. Appareil ECG selon une des revendications précédentes, **caractérisé en ce qu'**il comporte des électrodes thoraciques de Wilson.

8. Appareil ECG selon une des revendications précédentes, **caractérisé en ce que** cet appareil ECG est développé pour enclencher l'alimentation électrique entre l'électrode placée au niveau de l'ouverture supérieure du thorax et la première électrode de jambe distale placée sur une jambe et pour mesurer l'impédance ou la variation de l'impédance en fonction du battement cardiaque entre l'électrode placée au niveau de l'ouverture supérieure du thorax et la deuxième électrode de jambe distale placée sur la deuxième jambe ou entre les première et deuxième électrodes de jambe distales.

9. Appareil ECG selon une des revendications précédentes, **caractérisé en ce que** l'électrode sur l'ouverture supérieure du thorax est configurée comme une double électrode (1, 3) sur un support commun (37, 37a).

10. Appareil ECG selon une des revendications précédentes, **caractérisé en ce qu'**un courant alternatif peut être injecté dans les électrodes à plusieurs fréquences, de préférence entre 1 et 10 kHz, entre 30 et 200 kHz et entre 200 et 1000 kHz.

11. Appareil ECG selon la revendication 10, **caractérisé en ce que** le dispositif de commutation (2) est conçu pour injecter du courant électrique sur une électrode placée aux extrémités inférieures et sur l'électrode placée au niveau de l'ouverture supérieure du thorax et que cet appareil ECG est développé pour mesurer l'impédance entre l'électrode placée au niveau de l'ouverture supérieure du thorax et une électrode placée au niveau de l'ouverture inférieure du thorax, l'électrode placée au niveau de l'ouverture inférieure du thorax étant de préférence configurée comme une électrode thoracique (V3-V6, V3r-V6r).

**12.** Appareil ECG selon une des revendications précédentes, **caractérisé en ce qu'**au moins certaines des électrodes sont configurées comme des électrodes à vide, un dispositif qui régule et/ou optimise la valeur de la dépression des électrodes d'aspiration en fonction d'un signal physique déterminé, p. ex. d'un signal mécanique, acoustique, électrique ou optique, étant prévu de manière facultative.

**13.** Appareil ECG selon une des revendications précédentes, **caractérisé en ce que** plusieurs courbes de mesure enregistrées par les électrodes sont superposées pour former une courbe de mesure type au moyen d'une unité de calcul.

**14.** Appareil ECG selon une des revendications précédentes, **caractérisé en ce que** cet appareil ECG est configuré pour mesurer la variation de l'impédance en fonction du battement cardiaque sur au moins deux segments corporels et pour calculer à partir de cela et facultativement en outre à partir d'une mesure concernant le volume extracellulaire, p. ex. du rapport entre l'eau extracellulaire et l'eau corporelle totale ou du rapport entre l'eau extracellulaire et l'eau intracellulaire, la performance cardiaque et le degré d'une insuffisance cardiaque et/ou un paramètre biochimique pour le stress biomécanique de cellules du muscle cardiaque et/ou une accumulation d'eau et/ou d'un des peptides natriurétiques atriaux, p. ex. de BNP, de NT-proBNP ou d'adrénomédulline.

**15.** Appareil ECG selon une des revendications précédentes, **caractérisé en ce qu'**il comprend une unité de calcul qui est configurée pour calculer une relation entre un paramètre concernant la masse graisseuse ou la masse hydrique totale (TBW) ou la "lean body mass" (LBM) par rapport à la masse corporelle totale, et un paramètre concernant l'eau extracellulaire (ECW), p. ex. le rapport ECW/TBW ou ECW/ICW à partir des impédances segmentaires à basses et hautes fréquences pour les segments proches du coeur et/ou éloignés du coeur ou pour le corps complet, et pour délivrer ce rapport en relation avec les valeurs normales et/ou une ligne de régression.

**16.** Appareil ECG selon une des revendications précédentes, **caractérisé en ce qu'**il comprend une mémoire (40a) pour enregistrer les données déterminées et calculées et qu'il possède une interface avec un écran et/ou une imprimante pour afficher sur un écran et/ou imprimer sur une imprimante les valeurs actuelles et les valeurs antérieures des paramètres électriques et physiques collectés dans le temps, sous la forme de chiffres ou de graphiques avec des champs normaux, lequel appareil ECG est de préférence configuré pour représenter graphiquement, lorsqu'il y a plusieurs phases de mesure, les résultats des phases de mesure graphiquement sous forme de tendance au cours d'une analyse individuelle et pour représenter graphiquement, lors d'une analyse réitérée ultérieurement, les phases de mesure des différentes analyses superposées avec une indication temporelle.

**17.** Appareil ECG selon une des revendications précédentes, **caractérisé en ce qu'**un corps d'électrode est équipé d'une connexion facilement détachable, p. ex. d'une connexion à bouton-pression (45) qui conduit à une électrode qui est configurée de préférence comme une électrode adhésive (46) ou une électrode de serrage (1) ou une électrode à bande élastique, le corps d'électrode pouvant être inséré dans une ventouse (15).

**18.** Appareil ECG selon une des revendications précédentes, **caractérisé en ce qu'**au moins une des électrodes de dérivation d'ECG (23) est équipée d'un manchon gonflable et d'un capteur mécanique (9) pour les vibrations mécaniques sous la forme d'un accéléromètre (9b), lequel capteur mécanique (9) est configuré pour être positionné sur une artère située au-dessous, ce capteur mécanique (9) étant de préférence placé dans ou sur une vessie remplie de liquide (42a).

**19.** Appareil ECG selon une des revendications précédentes, **caractérisé en ce que** des branches reliées élastiquement d'une électrode sont présentes, parmi lesquelles une branche pour l'alimentation électrique (39a) et une branche faisant office de branche pour l'ECG et l'électrode de mesure d'impédance (39b), la distance des deux branches lorsqu'elles sont posées sur le corps étant conçue pour servir de conducteur ionique (40).

Fig. 1

Fig. 2

EP 2 958 489 B1

Fig. 3 a

Fig 3 b

Fig 4

Fig. 5

Fig. 6

Fig. 7

Fig. 8

Fig. 9

Fig. 10

Fig. 11

Fig. 12

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- AT 502921
- US 4628939 A, Little
- US 20010030077 A, Watson
- US 7110804 B
- WO 2006020764 A3
- WO 2008031030 A, Bartnik
- US 20050033190 A, Bauer
- US 20050273015 A, Bauer
- US 20090227886 A, Bauer
- US D675738 S
- WO 2006063255 A2, Bernstein
- US 20130096448 A, Brooks
- US 8521264 B
- US 20100324404 A
- US 6339722 B, Heethaar
- US 6560481 B, Heethaar
- US 7904141 B, Osypka
- US 4807638 A, Sramek
- WO 8903656 A1
- EP 2319411 A2
- WO 2007045006 A1
- EP 1275342 A2
- WO 2004030535 A1
- US 2005273015 A1
- US 2011245688 A1
- US 4646747 A

**In der Beschreibung aufgeführte Nicht-Patentliteratur**

- **BERTRAND CA et al.** *Circulation,* 1956, vol. 8, 49-57
- **WATT ; BURRUS.** *J Appl Physiol,* 1976, vol. 40, 171-176
- *J Appl Physiol,* 1976, vol. 40, 171-176
- **CHAITMAN et al.** *Circulation,* 1978, vol. 57, 71-79
- *IEEE Trans Biomed Eng,* 1983, vol. 30, 387-91
- *Eur J Appl Physiol,* 2003, vol. 89, 384-86
- **ZHU F et al.** *J Appl Physiol.,* 2006, vol. 100, 717-24